(19) Europäisches Patentamt
European Patent Office
Office européen des brevets

(11) EP 4 059 988 B1

(12) **EUROPEAN PATENT SPECIFICATION**

(45) Date of publication and mention of the grant of the patent:
**07.05.2025 Bulletin 2025/19**

(21) Application number: **21162711.2**

(22) Date of filing: **16.03.2021**

(51) International Patent Classification (IPC):
***C08G 65/40*** *(2006.01)* ***B01D 71/80*** *(2006.01)*
***B01J 41/13*** *(2017.01)* ***B32B 27/30*** *(2006.01)*
***C07D 211/22*** *(2006.01)* ***C08G 75/23*** *(2006.01)*
***C08J 5/22*** *(2006.01)* ***H01M 8/00*** *(2016.01)*

(52) Cooperative Patent Classification (CPC):
**C08G 75/23; B01D 71/38; B01D 71/68; B01D 71/72; B01D 71/80; B01D 71/82; B32B 27/12; B32B 27/30; C07D 211/22; C07D 471/10; C08G 65/40; C08G 65/4056; C08J 5/2256; C25B 13/08; H01M 8/1027;** (Cont.)

(54) **LONG-TERM ANION-CONDUCTING COMPOUND, ITS PREPARATION AND ITS USE IN ELECTROCHEMISTRY**

VERBINDUNG MIT LANGFRISTIGER IONENLEITUNG, DEREN HERSTELLUNG UND DEREN VERWENDUNG IN DER ELEKTROCHEMIE

COMPOSÉ CONDUCTEUR D’ANIONS À LONG TERME, SA PRÉPARATION ET SON UTILISATION EN ÉLECTROCHIMIE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**21.09.2022 Bulletin 2022/38**

(73) Proprietor: **Evonik Operations GmbH**
**45128 Essen (DE)**

(72) Inventors:
- **MALJUSCH, Artjom**
  **44866 Bochum (DE)**
- **CONRADI, Oliver**
  **40545 Düsseldorf (DE)**
- **RÖGL, Harald**
  **4702 Wallern an der Trattnach (AT)**
- **LUPPI, Gianluigi**
  **40479 Düsseldorf (DE)**

(74) Representative: **Evonik Patent Association**
**c/o Evonik Industries AG**
**IP Management**
**Postcode 84/339**
**Rodenbacher Chaussee 4**
**63457 Hanau (DE)**

(56) References cited:
**EP-A1- 3 770 201** **WO-A1-2017/172824**
**WO-A2-2019/068051** **CN-A- 104 829 813**
**CN-A- 104 829 814** **CN-A- 106 750 303**
**CN-A- 109 762 190** **CN-A- 110 294 845**

- **LI SU ET AL: "Anion conductive piperidinium based poly (ether sulfone): Synthesis, properties and cell performance", JOURNAL OF MEMBRANE SCIENCE, ELSEVIER BV, NL, vol. 594, 13 September 2019 (2019-09-13), XP085877527, ISSN: 0376-7388, [retrieved on 20190913], DOI: 10.1016/J.MEMSCI.2019.117471**
- **WANG FEN ET AL: "Synthesis and property of novel anion exchange membrane based on poly(aryl ether sulfone)s bearing piperidinium moieties", JOURNAL OF MEMBRANE SCIENCE, ELSEVIER BV, NL, vol. 591, 1 August 2019 (2019-08-01), XP085764659, ISSN: 0376-7388, [retrieved on 20190801], DOI: 10.1016/ J.MEMSCI.2019.117334**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)
**H01M 8/1067;** B01D 61/422; B01D 61/44;
B01D 2325/16; B32B 2457/10; B32B 2457/18;
C08J 2371/10; C08J 2381/06; C25B 1/04;
H01M 8/1069; H01M 8/18; H01M 2008/1095;
Y02E 60/50

## Description

The present invention provides compounds, especially polymeric compounds, a process for preparation thereof and for the use of these compounds. Intended use is in field of electrochemistry. Anion-conducting properties of disclosed compounds making this material suitable for the preparation of anion-conducting membranes.

One important example for an electrochemical process is electrolysis of water to gain molecular hydrogen and molecular oxygen. The electrochemical aggregate used to perform such process is called electrolyzer. Such electrolyzer typically comprise many electrochemical cells. Each electrochemical cell comprises two compartments, each equipped with one gas evolving electrode and a membrane separating both compartments. To enable electrolytic splitting of water, the membrane needs to be conductive for ions (cations or anions), while almost impermeable for hydrogen and oxygen gas. Compounds discussed herein are intended to compose such membranes.

The membrane employed in electrochemical cells need to be electrically non-conductive, as otherwise an electrical short circuit between both electrodes occurs. Thus, from an electrical point of view, the membrane needs to be electrically isolating. However, from an electrochemical point of view, membranes in cells need to have a low ohmic resistance for ions passing the membrane from one compartment to the other one. The ability to let ions pass while electrons are isolated, is called ionic conductivity. In field of water electrolysis, anion conductivity of used anion-conducting membrane is of great importance.

Efficiency of electrochemical cells are strongly dependent of ionic conductivity of the membrane: If the ionic conductivity is low, a higher amount of electrical power must be consumed to generate desired amount of hydrogen due to ohmic loss leading to heat generation. Hence, providing a material with high ionic conductivity is a major target in membrane engineering.

Ionic conductivity is an intrinsic property of the membrane material. Due to harsh conditions within the electrochemical cell, the molecular structure of membrane material is subjected to chemical and electrochemical degradation. As a result, the ionic conductivity of membrane material degrades over time and process efficiency decreases leading to increasing consumption of electrical power.

Thus, a membrane with high initial ionic conductivity is worthless if ionic conductivity gets lost fast in short period of time.

Several anion exchange membranes for water electrolysis are commercially available. A market overview has been compiled by Henkensmeier et al.:

Henkensmeier, Dirk and Najibah, Malikah and Harms, Corinna and Žitka, Jan and Hnát, Jaromir and Bouzek, Karel (2020) Overview: State-of-the Art Commercial Membranes for Anion Exchange Membrane Water Electrolysis. Journal of Electrochemical Energy Conversion and Storage, 18 (2), 024001. American Society of Mechanical Engineers (ASME). DOI: 10.1115/1.4047963 ISSN 2381-6872

In section 3.7, Henkensmeier et al. already outlined the demand for long-term stability, but they are not able to identify membranes lasting over several 1000 hours.

An example for a commercially available anion exchange membrane is the product called fumasep® FAA-3-50, produced by FUMATECH BWT GmbH, 74321 Bietigheim-Bissingen, DE. According to Henkensmeier et al., this membrane is based on a polyaromatic polymer with ether bonds in the main chain and quaternary ammonium groups attached to the main chain. Own assessments of inventors confirmed that this membrane completely disintegrates after 120 hours in 2 M KOH at 80°C.

CN 104829814 A discloses a polymer containing a quaternized piperidine group. This polymer is used for preparing an anion exchange membrane as well. Long-term stability has not been assessed in CN 104829814 A.

A preparation method for a tertiary amine type polyarylether sulfone (ketone) polymer resin is known from CN 110294845 A. This polymer is used for preparing an anion exchange membrane. Long-term stability has not been assessed in this CN 110294845 A, but hereinafter: Polymer prepared using a tertiary amine bisphenol monomer (MPDDP) as described in CN 110294845 A disintegrated into several small pieces after 1000 h in 2M KOH at 80°C.

An anion conducting, piperidine containing polyether sulfone based material for anion exchange membranes has been prepared by Su Li et al.:

Su Li, Haibo Zhang, Kaiqi Wang, Fan Yang, Yuntao Han, Yirong Sun, Jinhui Pang, Zhenhua Jiang: Anion conductive piperidinium based poly (ether sulfone): Synthesis, properties and cell performance. Journal of Membrane Science, Volume 594, 2020, 117471, ISSN 0376-7388, https://doi.org/10.1016/j.memsci.2019.117471.

An anion exchange membrane made from a polyarylether sulfone piperidine structure has been developed by Fen Wang et al.:

Fen Wang, Boxin Xue, Shengyang Zhou, Jifu Zheng, Shenghai Li, Suobo Zhang, Tauqir A. Sherazi: Synthesis and property of novel anion exchange membrane based on poly(aryl ether sulfone)s bearing piperidinium moieties. Journal of Membrane Science, Volume 591, 2019 117334, ISSN 0376-7388, https://doi.org/10.1016/j.memsci.2019.117334.

Yet another polyarylether anion exchange membrane material is disclosed in CN 109762190 A.

A polymeric anion-conducting material suitable for preparing a membrane to be used in electrolyzers is known

from WO 2019/076860 A1. This material is characterized by at least one imidazole and/or imidazolium unit. This material shows good ionic conductivity, but long-term behavior is not assessed in WO 2019/076860 A1.

International patent application WO 2021/013694 A1 and European patent application EP 3770201 A1 relate to a polymeric anion-conducting membrane made from a compound containing at least one unit of the formula (0a)

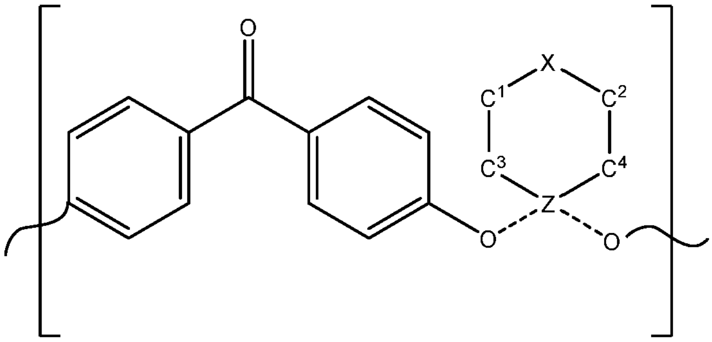

(0a)

with X being a structure element comprising a nitrogen atom with a positive charge bonded to $C^1$ and $C^2$ and bonded via two bonds to one or two hydrocarbon radical(s) comprising 1 to 12, preferably 1 to 6, more preferably 1 or 5 carbon atoms and Z being a structure element comprising a carbon atom being bonded to $C^3$ and $C^4$ and at least one aromatic 6-ring directly bonded to one of the oxygen atoms, wherein the aromatic rings might be substituted with one or more halogen and/or one or more $C_1$- to $C_4$- alkyl radicals.

As shown hereinafter, this material already achieves very high ionic conductivity which is upheld for a notable time: After 2000 h it in 2M KOH at 80°C it still possesses more than 85 % of its initial ionic conductivity.

However, demands regarding long-term stability becoming more and more important as sustainability is nowadays not only a matter of energy efficiency, but rather a matter of material sufficiency.

The object of the present invention is therefore to provide material having an ionic conductivity that is stable over long period of time.

The currently undisclosed European patent application 21152487.1 points in another direction as it focusses on mechanical stability. Said undisclosed application concerns to a compound that is characterized by at least one unit of the formula (0b)

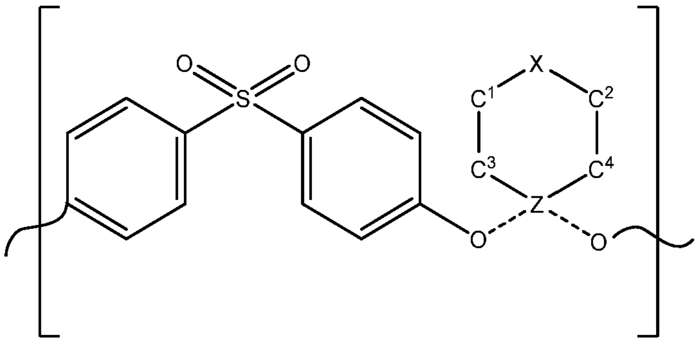

(0b)

with X being a structure element comprising at least one nitrogen atom with a positive charge bonded to $C^1$ and $C^2$ and bonded via two bonds to one or two hydrocarbon radical(s) comprising 1 to 12, preferably 1 to 6, more preferably 1 or 5 carbon atoms and Z being a structure element comprising a carbon atom being bonded to $C^3$ and $C^4$ and at least one aromatic 6-ring directly bonded to one of the oxygen atoms, wherein said aromatic 6-ring is substituted in position 3 and 5 with the same or different alkyl group having from 1 to 4 carbon atoms, preferably being a methyl, iso-propyl or tert-butyl group, more preferably being a methyl group. In preferred embodiments the structure element X of formula (0b) comprises positively charged nitrogen atoms being connected with a single aliphatic chain to adjacent structure elements.

Same inventors have now found improved compounds with slower decrease of ionic conductivity over time.

Thus, recently found compounds solved aforesaid problem and are consequently subject matter of the present invention. These inventive compounds are described hereinafter and in the claims as well.

Inventive compounds containing at least one unit of the formula (I)

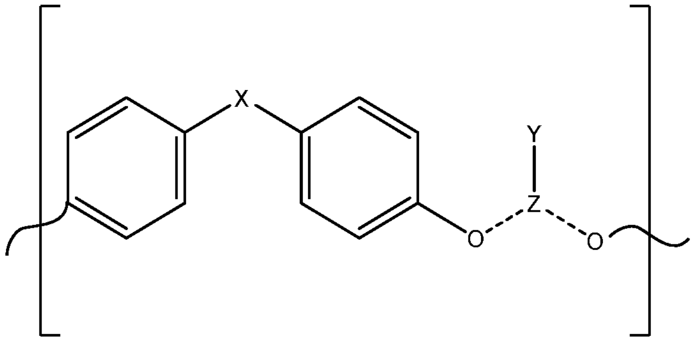

(I)

wherein X being a ketone or sulfone group, wherein Z being a structure element comprising at least one tertiary carbon atom and at least one aromatic 6-ring directly bonded to one of the oxygen atoms, and wherein Y being a structure element comprising at least one nitrogen atom with a positive charge and Y being bonded to said tertiary carbon atom of Z.

Hence, compared to prior art compounds mentioned above, inventive compounds are characterized by at least one tertiary carbon atom within structure element Z, to which the structure element Y is bonded to.

Compared to prior art material of similar chemical nature, inventive compounds achieve a better long-term stability of ionic conductivity.

According to a preferred embodiment of the invention the compound is represented by formula (Ia) or (Ib)

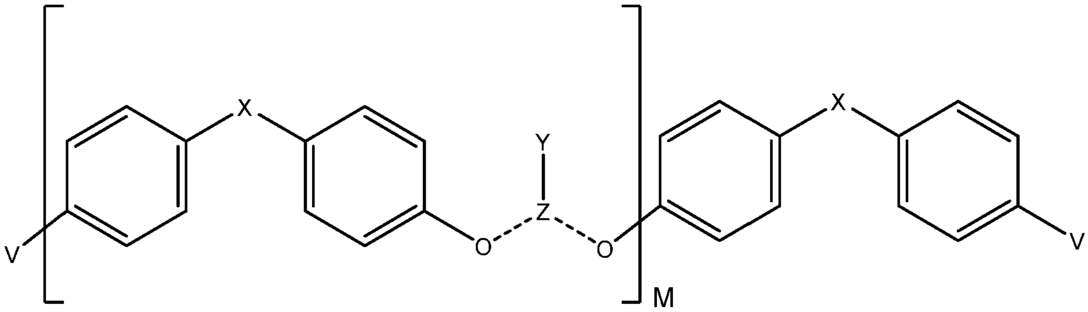

(Ia)

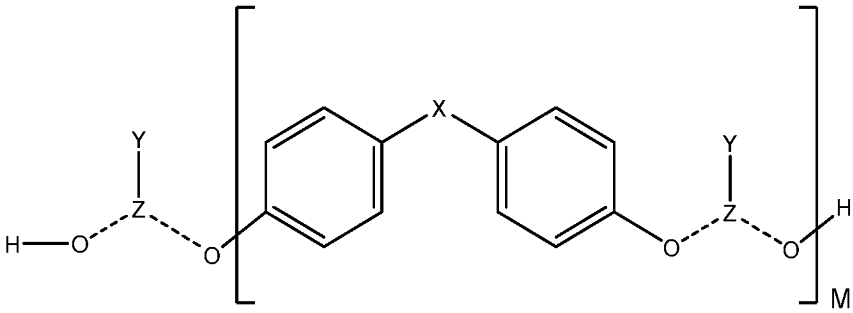

(Ib)

wherein V represents same or different halogen. Preferably, V being Fluor and M being an integer from 1 to 1000. Most preferred, M being an integer from 5 to 500. If M being 1, compound is considered monomeric. If M is above 1, inventive compound is considered polymeric.

According to a preferred embodiment of the invention, structure element Y represents a unit of formula (IIa), (IIb) or (IIc)

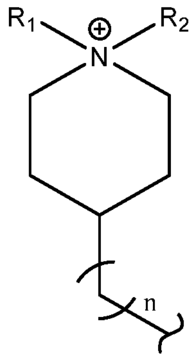

(IIa)

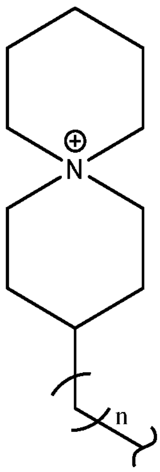

(IIb)

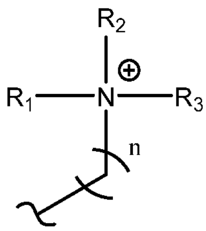

(IIc)

with n representing number of carbon atoms in the aliphatic chain and n being from 0 to 9. Preferably, n being from 0 to 5; $R_1$, $R_2$ and $R_3$ being the same or different alkyl group having from 1 to 9 carbon atoms, preferably $R_1$, $R_2$ and $R_3$ each being a methyl group.

According to a preferred embodiment of the invention, the structure element Y present in the compound represents in more than 5 %, preferably in more than 50 %, and most preferred in more than 90 % of its occurrence a unit of formula (IIa), (IIb) or (IIc). The occurrence can be determined for example by classical $^1$H-NMR performed accordingly to 01/2005:20233 (EUROPEAN PHARMACOPOEIA 5.0. 2.2.33. Nuclear magnetic resonance spectrometry) at room temperature in trideuterio(trideuteriomethylsulfonyl)methane (DMSO-d6) as solvent. The occurrence can be calculated via integration of the area of corresponding signal and comparison of normalized area of corresponding signal (peak) with the number of corresponding protons in a target unit.

According to a further preferred embodiment of the invention, structure element Z represents a unit of formula (III)

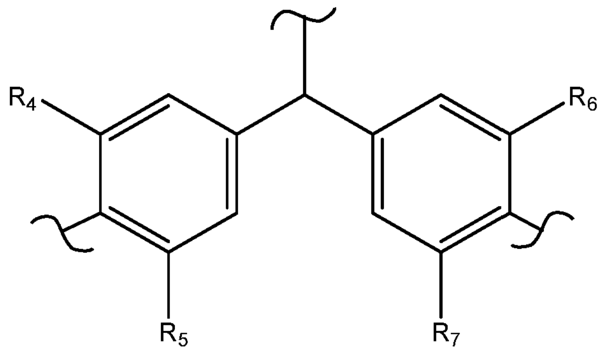

(III)

with $R_4$, $R_5$, $R_6$ and $R_7$ being the same or different alkyl group having from 1 to 4 carbon atoms, $R_4$, $R_5$, $R_6$ and $R_7$ each preferably being a methyl, iso-propyl or tert-butyl group, more preferably $R_4$, $R_5$, $R_6$ and $R_7$ each being a methyl group.

Six preferred embodiments of inventive compound are represented by at least one of formulas (IVa) to (IVf):

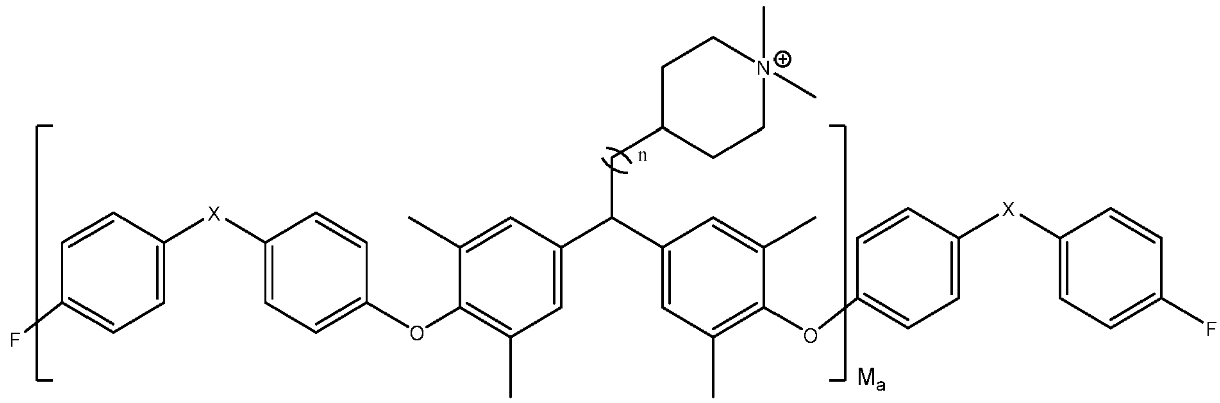

(IVa)

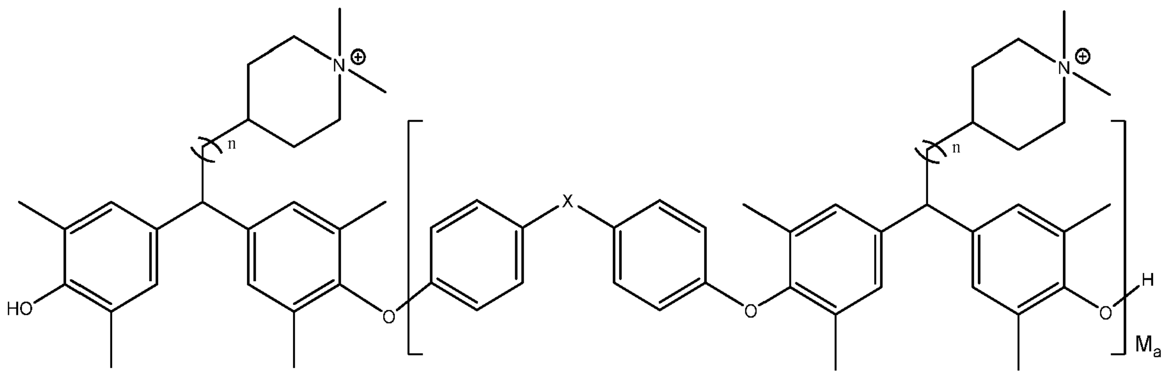

(IVb)

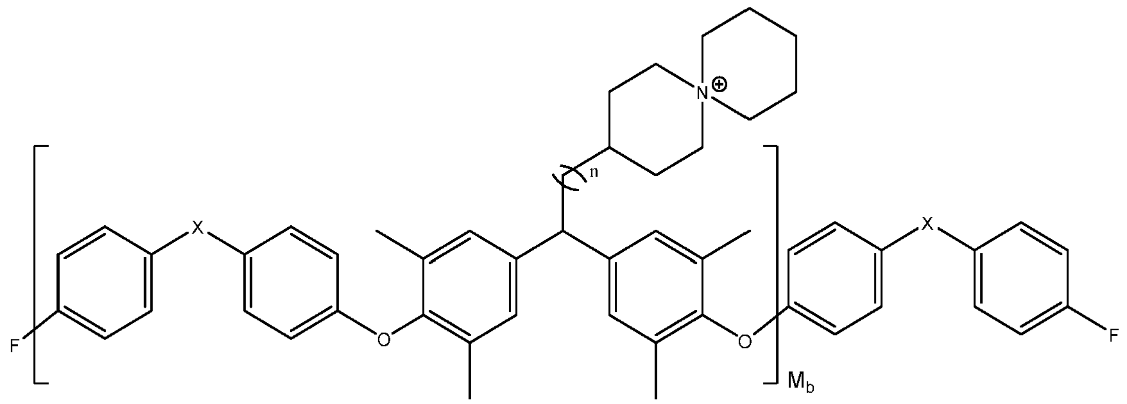

(IVc)

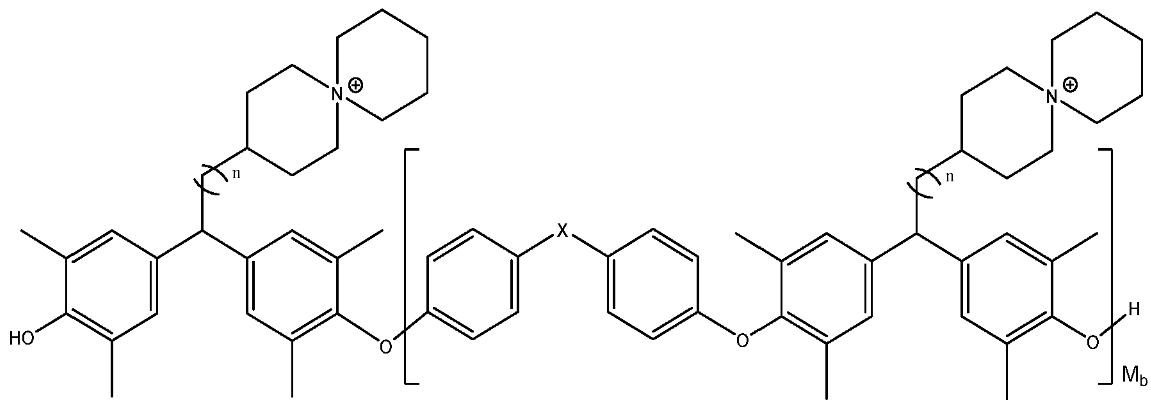

(IVd)

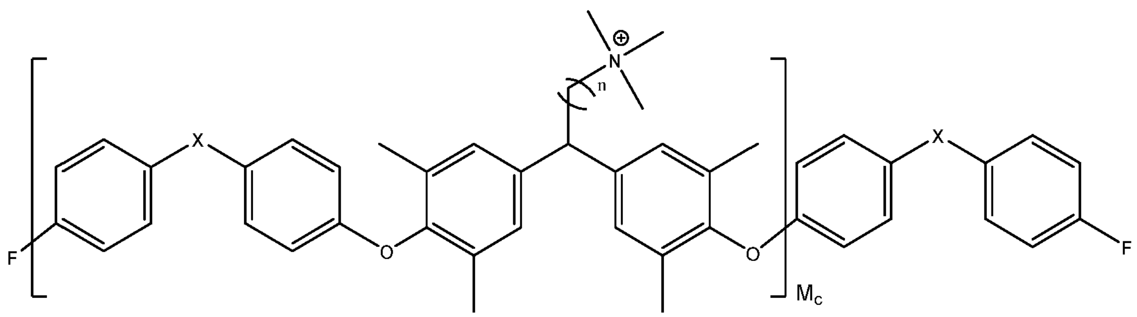

(IVe)

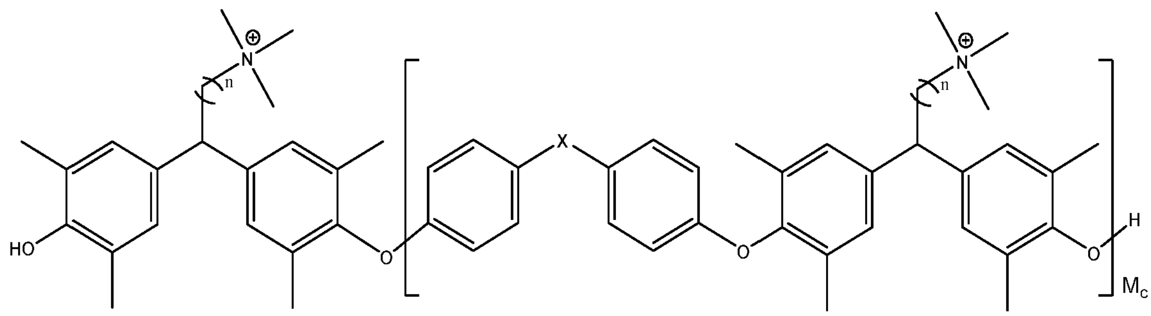

(IVf)

In formulas (IVa) to (IVf) n representing number of carbon atoms in the aliphatic chain and n being from 0 to 9,

preferably from 0 to 5 and with $M_a$, $M_b$ and $M_c$ each being an integer of from 1 to 1000, preferably $M_a$, $M_b$, and $M_c$ each being an integer of from 5 to 500.

As derivable from the formulae (I), (Ia), (Ib), (IVa) to (IVf) and (Va) to (Vd) and related definitions, all inventive compounds comprising an aromatic 6-ring which is directly bonded to one of the oxygen atoms, which is substituted in position 3 and 5 with the same or different alkyl group having from 1 to 4 carbon atoms.

According a first variant of the invention, said aromatic 6-ring is further substituted with one or more halogen and/or one or more $C_1$- to $C_4$- alkyl radicals. Preferred alkyl radicals are selected from the list consisting of methyl, iso-propyl, and tert-butyl. The most preferred alkyl is methyl.

According to a second, preferred variant of the invention, said aromatic 6-ring is free of any further substitution with one or more halogen and/or one or more $C_1$- to $C_4$- alkyl radicals. Precursor materials for preparing such compounds are cheaper. Thus, preparation and final compound is less cost intensive.

Yet another object of the present invention is to provide a process for preparing inventive compounds.

This object is solved by a process comprising the following steps:

a) providing a first educt, wherein said first educt comprises at least one compound of the formula (VIa) or (VIb), where V represents same or different halogen, preferred Fluor,

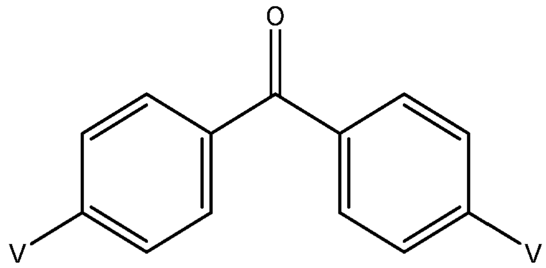

(VIa)

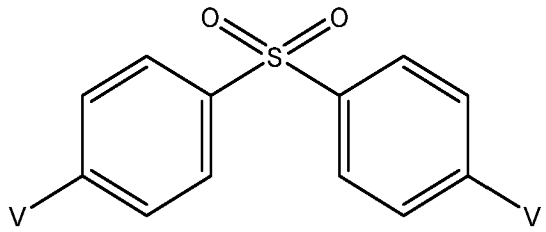

(VIb)

b) providing a second educt, wherein said second educt comprises at least one compound selected from formulas (VIIa), (VIIb), (VIIc) or (VIId)

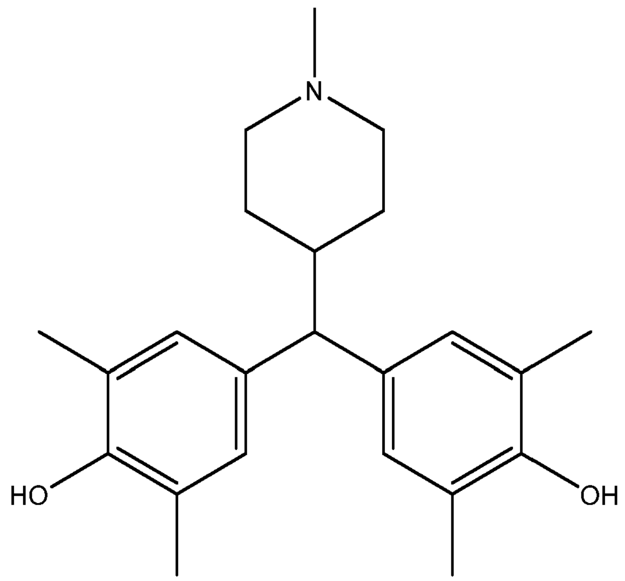

(VIIa)

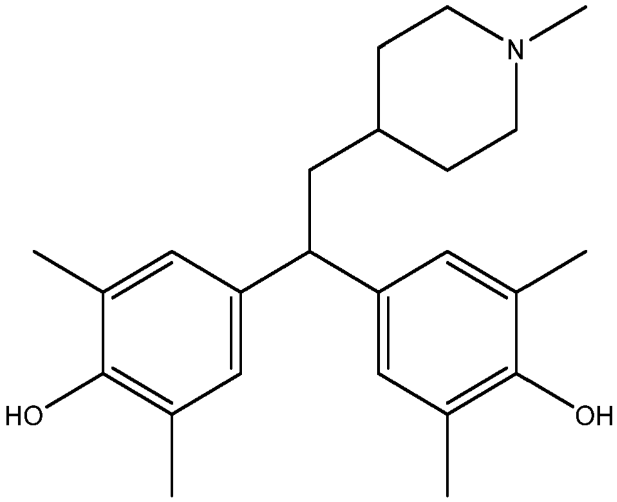

(VIIb)

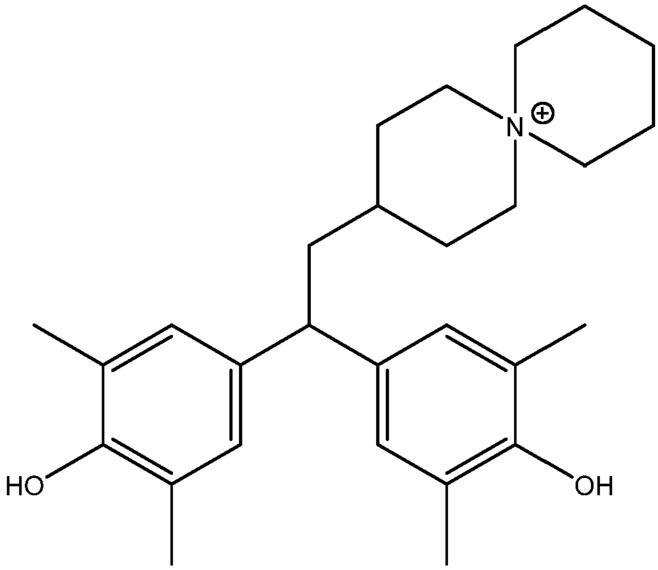

(VIIc)

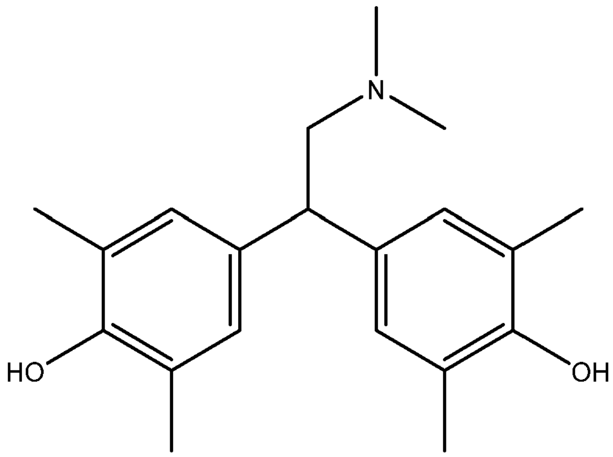

(VIId)

c) Reacting said first educt with said second educt;
d) Obtaining at least one inventive compound.

Such process is quite simple to conduct and yields desired compounds. Compounds (VIa) and (VIb) - precursors within first educt - are commercially available. Compounds according formulas (Vila), (VIIb), (VIIc) or (VIId) - precursors

within second educt - can be prepared by means of classical organic chemistry. Reaction routines are derivable from examples 1, 8, 15 and 18 respectively.

Preferably, this reaction step is carried out at a temperature of from 100 °C to 300 °C, more preferably at a reaction temperature of from 125 °C to 175 °C. Most preferably the reaction step is carried out at a temperature where the reaction mixture is boiling, preferably while stirring. The reaction step is most preferably carried out under an inert gas atmosphere, preferably a nitrogen atmosphere. At the top of the reaction vessel, any water formed is preferably removed.

The reaction step is preferably carried out in the presence of a base like KOH, NaOH, $K_2CO_3$ or $Na_2CO_3$. Preferred base is $K_2CO_3$.

The reaction step is carried out in the presence of an organic solvent. Preferred solvents are selected from the list consisting of N-Methyl-2-pyrrolidone (NMP), Dimethyl sulfoxide (DMSO), N,N-Dimethylformamide (DMF) and N,N-Dimethylacetamide (DMAC). Preferably N,N-Dimethylacetamide is used as a solvent.

Preferably the process according to the invention comprises a step where an alkylating reagent, preferably a methylating reagent, is used. Preferred methylating agent used is iodomethane or chloromethane. If alkylation is performed, this step is chronologically situated before obtaining inventive compound. In this case, prepared inventive compound is an alkylated one.

As set out above all inventive compounds comprising an aromatic 6-ring which is directly bonded to one of the oxygen atoms, which is substituted in position 3 and 5 with the same or different alkyl group having from 1 to 4 carbon atoms. According a first variant of the invention, said aromatic 6-ring is further substituted with one or more halogen and/or one or more $C_1$- to $C_4$- alkyl radicals. According to a second, preferred variant of the invention, said aromatic 6-ring is free of any further substitution with one or more halogen and/or one or more $C_1$- to $C_4$- alkyl radicals.

To realize both variants of inventive compounds during the course of inventive preparation method, precursors - namely compound of formula (VIa) or (VIb) or (VIIa) or (VIIb) or (VIIc) or (VIId) - are selected accordingly.

According to the first variant the aromatic rings of these compound (precursors) are further substituted with one or more halogen and/or one or more $C_1$- to $C_4$- alkyl radicals, whereby alkyl radicals selected from the list consisting of methyl, iso-propyl, and tert-butyl are preferred ones while methyl is the most preferred alkyl.

According to the second the aromatic rings of these compounds (precursors) are free of any further substitution with one or more halogen and/or one or more $C_1$- to $C_4$- alkyl radicals.

The compounds of the present invention might be used for different purposes. Preferably the compounds of the present invention are polymers and are used as anion-conducting membranes or at least for the production of anion-conducting membranes. Such use is a further object of present invention.

Within such membranes inventive compounds serve as separation active material due to their long-term stable anion-conducting properties, while being very gas tight. Beside inventive compounds mentioned membranes may comprise further materials, for instance porous support, e.g. a fabric or non-woven material.

Thanks to designed properties of the component disclosed herein, an anion-conducting membrane comprising such material may be employed in an electrochemical cell. Thus, another embodiment of the invention is an electrochemical cell having an anion-conducting membrane, wherein said anion-conducting membrane comprises inventive compound.

Certain mechanical and chemical stability of present compounds makes this material suitable for employment in electrolyzers or a fuel cells or redox flow batteries. Thus, each a preferred embodiment of inventive electrochemical cell is an electrolyzer, a fuel cell or a redox flow battery.

An electrochemical process performed by means of an inventive electrochemical cell is another embodiment of the invention.

Preferably, said electrochemical process is an electrolysis or an electrodialysis or an electrochemical process taking place during operation of a fuel cell or an electrochemical process taking place during operation of a redox flow battery.

Further details of present invention are derivable from the examples and accompanying figures. The latter show:

**Figure 1:** $^1$H-NMR spectrum of monomer (VIIa)
**Figure 2**: $^1$H-NMR spectrum of quaternized piperidine containing polymer from Example 3
**Figure 3**: $^1$H-NMR spectrum of quaternized piperidine containing polymer from Example 6
**Figure 4:** $^1$H-NMR spectrum of monomer (VIIb)
**Figure 5:** $^1$H-NMR spectrum of quaternized piperidine containing polymer from Example 10
**Figure 6:** $^1$H-NMR spectrum of quaternized piperidine containing polymer from Example 13
**Figure 7:** $^1$H-NMR spectrum of monomer (VIIc)
**Figure 8:** $^1$H-NMR spectrum of spiro containing polymer from Example 16
**Figure 9:** $^1$H-NMR spectrum of monomer (VIId)
**Figure 10:** $^1$H-NMR spectrum of quaternized piperidine containing polymer from Example 20
**Figure 11:** $^1$H-NMR spectrum of quaternized piperidine containing polymer from Example 23

**Examples:**

**Example 1: Synthesis of piperidine containing monomer (VIIa) (part of invention)**

Synthesis of piperidine containing monomer (VIIa) was performed in two steps accordingly to reaction scheme 1 (step 1) and reaction scheme 2 (step 2).

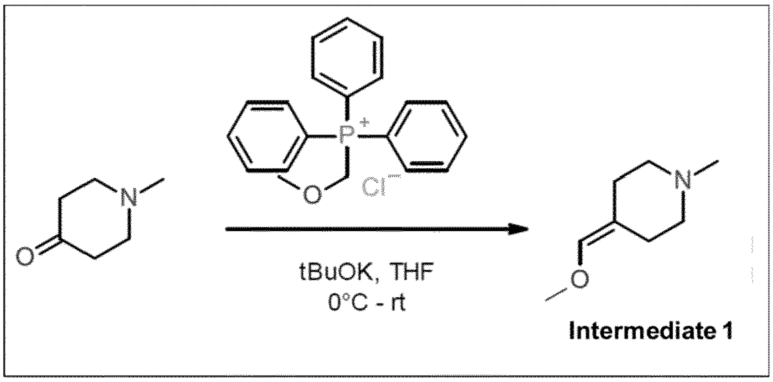


Reaction scheme 1 (step1)

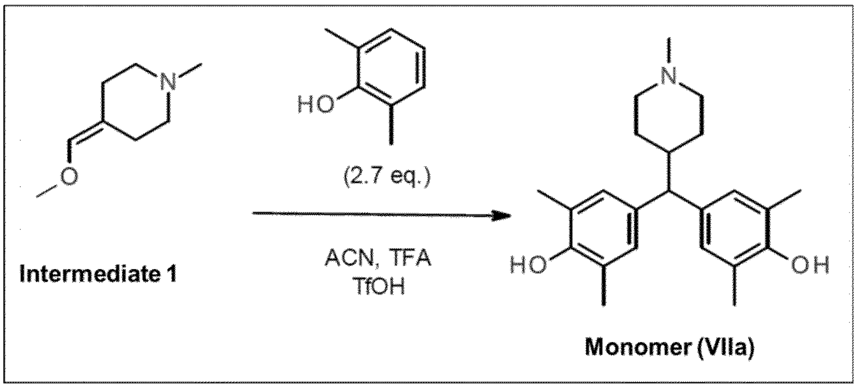


Reaction scheme 2 (step 2)

A mixture of 227.2 g (662.778 mmol) of Methoxymethyl-triphenyl-phosphine chloride in 1.3 L of dry THF was placed in 6 L three-neck round-bottom flask equipped with thermometer, dropping funnel, mechanical stirrer, ice bath, $N_2$ atmosphere and cooled down to 0°C. A solution of 99.16 g (883.704 mmol) of potassium tert-butoxide in 900 mL of dry Tetrahydrofuran (THF) was added dropwise and reaction mixture was stirred at room temperature for 30 minutes. After 30 minutes, the reaction mixture was cooled down to 0°C and a solution of 50 g (441.852 mmol) of 1-Methylpiperydin-4-one in 900 mL of dry THF was added dropwise to the reaction mixture. The mixture was stirred at room temperature overnight. The reaction mixture was divided in two batches (batch 1 and batch 2). Batch 1 was poured onto 1 L of ice water and extracted with 5 x 200 ml of Dichloromethane (DCM). Combined organic layer was washed with brine, dried over $MgSO_4$ and solvent was removed under vacuum to give 45.25 g of crude material. Column chromatography with silica as stationary phase and DCM / Methanol (+1 % $NH_3$) as mobile phase were used for purification to give 24.89 g of Intermediate 1 (yield 55 %).

In a three-necked-flask (500 mL) equipped with thermometer, dropping funnel, mechanical stirrer and $N_2$ atmosphere 16 g. (0.113 mol) of Intermediate 1 were placed. A solution of 37.4 g (0.306 mol) of 2,6-Dimethylphenol in 170 mL acetonitrile was added dropwise at 10°C. Additionally, 26 mL (0.34 mol) of trifluoroacetic acid (TFA) were added dropwise, followed by dropwise addition of 12 mL (0.136 mol) of trifluoromethanesulfonic acid. The resulting solution was stirred at room temperature overnight. The reaction mixture was evaporated under vacuum. The crude residue was treated with diethyl ether ($Et_2O$, 300 mL) and stirred overnight at room temperature. The solid was filtered, washed with $Et_2O$ (100 mL) and dried under high vacuum at 45°C. Obtained TFA-salt of product was converted to free base by dissolution of it in 90 mL of acetonitrile, followed by addition 6 mL of water and basification with 10 % aq. ammonia to pH~8. As result white precipitate was formed. The precipitate was collected by filtration, washed with 10 mL of water and dried under vacuum. Obtained white solid was dissolved in 1 L methanol, concentrated under vacuum until the solution becomes

cloudy and stored at room temperature. Crystallized solid was filtered of by vacuum filtration and dried under vacuum. Normal phases column chromatography was used for purification to give 24.97 g (yield 62 %) of piperidine containing monomer (VIIa). Chemical structure of monomer (VIIa) was confirmed by $^1$H-NMR; $^1$H-NMR spectrum is given in Figure 1. trideuterio(trideuteriomethylsulfonyl)methane (DMSO-d6) was used as solvent.

**Example 2: Synthesis of piperidine containing polymer (part of invention)**

Synthesis was performed in a 500 mL three-necked flask with oil bath, mechanical stirrer, a packed column with distillation head cooler with adjustable return ratio and condensate removal. At the beginning of synthesis 10.61 g (0.03 mol) of piperidine containing monomer (VIIa) from Example 1, 6.55 g (0.03 mol) of 4,4'-Difluorobenzophenon (VIa), 165 mL of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 14.64 g (91.8 %).

**Example 3: Quaternization of piperidine containing polymer from Example 2 (part of invention)**

10 g of the polymer from Example 2 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized piperidine containing polymer from Example 3 was confirmed by $^1$H-NMR; $^1$H-NMR spectrum is given in Figure 2. DMSO-d6 was used as solvent.

**Example 4: Membrane casting of piperidine containing polymer from Example 3 (part of invention)**

The solution of the quaternized polymer from Example 3 was directly used for preparation of the membrane. The required amount of polymer solution was taken up with a syringe and applied directly through a 1 $\mu$m Polytetrafluoroethylene (PTFE) filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 5: Synthesis of piperidine containing polymer (part of invention)**

Synthesis was performed in a 500 mL three-necked flask with oil bath, mechanical stirrer, a packed column with distillation head cooler with adjustable return ratio and condensate removal. At the beginning of synthesis 10.61 g (0.03 mol) of piperidine containing monomer (VIIa) from Example 1, 7.63 g (0.03 mol) of 4,4'-Difluordiphenylsulfon (VIb), 165 mL of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 15.21 g (89.3 %).

**Example 6: Quaternization of piperidine containing polymer from Example 5 (part of invention)**

10 g of the polymer from Example 5 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized piperidine containing polymer from Example 6 was confirmed by $^1$H-NMR; $^1$H-NMR spectrum is given in Figure 3. DMSO-d6 was used as solvent.

**Example 7: Membrane casting of piperidine containing polymer from Example 6 (part of invention)**

The solution of the quaternized polymer from Example 3 was directly used for preparation of the membrane. The

required amount of polymer solution was taken up with a syringe and applied directly through a 1 µm PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 8: Synthesis of piperidine containing monomer (VIIb) (part of invention)**

Synthesis of piperidine containing monomer (VIIb) was performed in three steps accordingly to reaction scheme 3 (step 1), reaction scheme 4 (step 2) and reaction scheme 5 (step 3).

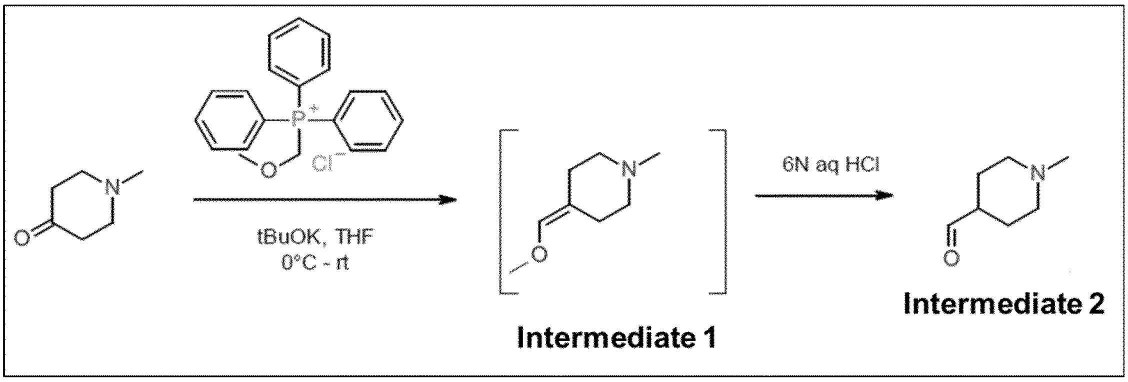


Reaction scheme 3 (step 1)

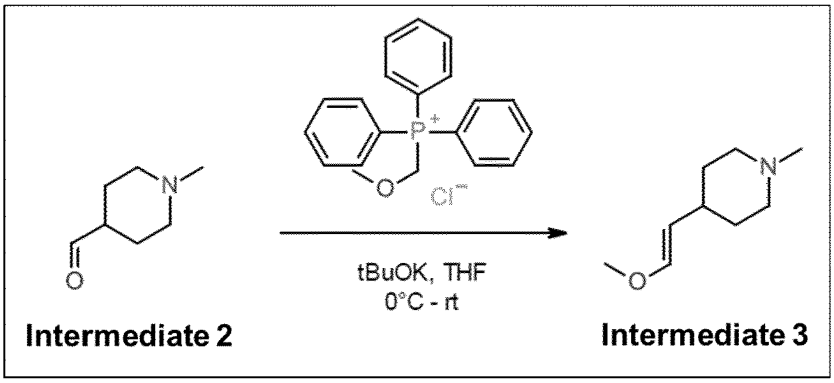


Reaction scheme 4 (step 2)

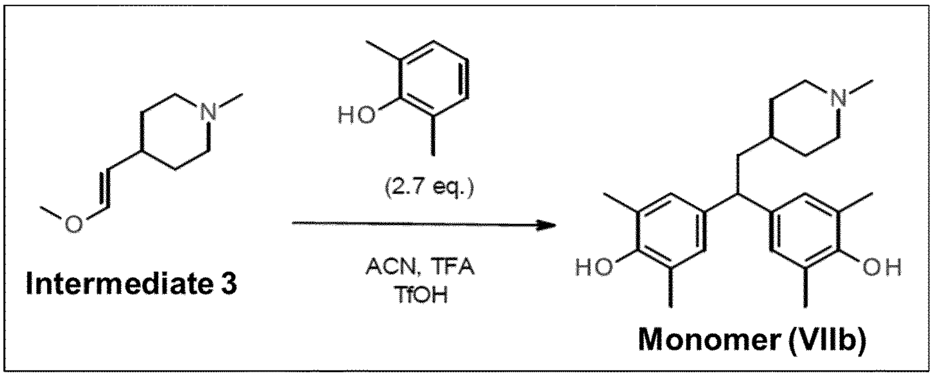


Reaction scheme 5 (step 3)

Aqueous solution of 6 N HCl was added to Batch 2 from Example 1 to pH =1. Reaction mixture was stirred with 1.5 L of $Et_2O$ for 30 minutes. Then organic phase was separated, and aqueous phase was extracted again with 1 L of $Et_2O$. Obtained aqueous phase was basified with 3 N NaOH to pH ~10 and extracted with DCM (3 x 1 L). Organic phase was

separated and concentrated to dryness to give 45.5 g of crude product (Intermediate 2).

In a four-necked-flask (6 L) equipped with thermometer, dropping funnel, mechanical stirrer, ice bath and $N_2$ atmosphere 259.84 g (0.758 mol) of Methoxymethyl-triphenyl-phosphine chloride was suspended in1.55 L of dry THF and cooled down to 0°C. To this suspension, a solution of 127.58 g (1.137 mol) of Potassium tert-butoxide in 1 L of THF was added dropwise. The resulting solution was stirred at room temperature for 1 hour. The reaction mixture was cooled down to 0°C and a solution of 45.5 g of crude product (Intermediate 2) in 780 mL of THF was added to the reaction mixture. The resulting reaction mixture was stirred at room temperature overnight. The reaction mixture was poured into 3 L of ice-cold water, extracted with 3 x 3 L of DCM until the wp showed no more product, combined organic phase was washed with brine, dried over $MgSO_4$ and concentrated under vacuum. Column chromatography with silica as stationary phase and DCM / Methanol (+1 % $NH_3$) as mobile phase were used for purification to give 52.89 g of Intermediate 3.

In a three-necked-flask (500 mL) equipped with thermometer, dropping funnel, mechanical stirrer, ice bath and $N_2$ atmosphere 47.5 g (0.306 mol) of Intermediate 3 were placed. A solution of 101 g (0.827 mol) of 2,6-Dimethylphenol in 230 mL acetonitrile was added dropwise at 10 °C. Additionally, 70 mL (0.919 mol) of TFA were added dropwise, followed by dropwise addition of 32 mL (0.367 mol) of trifluoromethanesulfonic acid and the resulting solution was stirred at room temperature overnight. Reaction mixture was concentrated to dryness in vacuum and obtained crude was stirred with 1 L of $Et_2O$. The $Et_2O$ filtrate was decanted and again 1 L of $Et_2O$ was added. The sticky solid was filtered, washed with $Et_2O$ (500 mL) and dried under vacuum. A brown solid was stirred in 300 mL ethyl acetate for 30 minutes, the off-white solid was filtered, washed with ethyl acetate (100 mL) and dried under high vacuum to give an off-white solid. Obtained TFA-salt of product was converted to free base by suspension it in 100 mL of water and neutralization of it with 200 mL of 25 % $NH_3$ leading to precipitation of piperidine containing monomer (VIIb) as a white solid. Obtained product was dried under vacuum overnight and 20.67 g of product were purified by normal phase column chromatography to give 12.13 g (yield 59 %) of piperidine containing monomer (VIIb). Chemical structure of monomer (VIIb) was confirmed by $^1$H-NMR; $^1$H-NMR spectrum is given in Figure 4. DMSO-d6 was used as solvent.

**Example 9: Synthesis of piperidine containing polymer (part of invention)**

Synthesis was performed in a 500 mL three-necked flask with oil bath, mechanical stirrer, a packed column with distillation head cooler with adjustable return ratio and condensate removal. At the beginning of synthesis 11.03 g (0.03 mol) of piperidine containing monomer (VIIb) from Example 8, 6.55 g (0.03 mol) of 4,4'-Difluorobenzophenon (VIa), 165 mL of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 15.14 g (92.4 %).

**Example 10: Quaternization of piperidine containing polymer from Example 9 (part of invention)**

10 g of the polymer from Example 9 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized piperidine containing polymer from Example 10 was confirmed by $^1$H-NMR; $^1$H-NMR spectrum is given in Figure 5. DMSO-d6 was used as solvent.

**Example 11: Membrane casting of piperidine containing polymer from Example 10 (part of invention)**

The solution of the quaternized polymer from Example 3 was directly used for preparation of the membrane. The required amount of polymer solution was taken up with a syringe and applied directly through a 1 $\mu$m PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 12: Synthesis of piperidine containing polymer (part of invention)**

Synthesis was performed in a 500 mL three-necked flask with oil bath, mechanical stirrer, a packed column with distillation head cooler with adjustable return ratio and condensate removal. At the beginning of synthesis 11.03 g (0.03 mol) of piperidine containing monomer (VIIb) from Example 8, 7.63 g (0.03 mol) of 4,4'-Difluordiphenylsulfon (VIb), 165 mL

of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 15.39 g (88.1%).

**Example 13: Quaternization of piperidine containing polymer from Example 12 (part of invention)**

10 g of the polymer from Example 12 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized piperidine containing polymer from Example 10 was confirmed by $^1$H-NMR; $^1$H-NMR spectrum is given in Figure 6. DMSO-d6 was used as solvent.

**Example 14: Membrane casting of piperidine containing polymer from Example 13 (part of invention)**

The solution of the quaternized polymer from Example 13 was directly used for preparation of the membrane. The required amount of polymer solution was taken up with a syringe and applied directly through a 1 μm PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 15: Synthesis of spiro containing monomer (VIIc) (part of invention)**

Synthesis of spiro containing monomer (VIIc) was performed in four steps accordingly to reaction scheme 6 (step 1), reaction scheme 7 (step 2), reaction scheme 8 (step 3) and reaction scheme 9 (step 4). Intermediate 4 was synthesized following exact same steps like by synthesis of monomer (VIIb) but starting with 1-Benzylpiperidin-4-one instead of 1-Methylpiperydin-4-one.

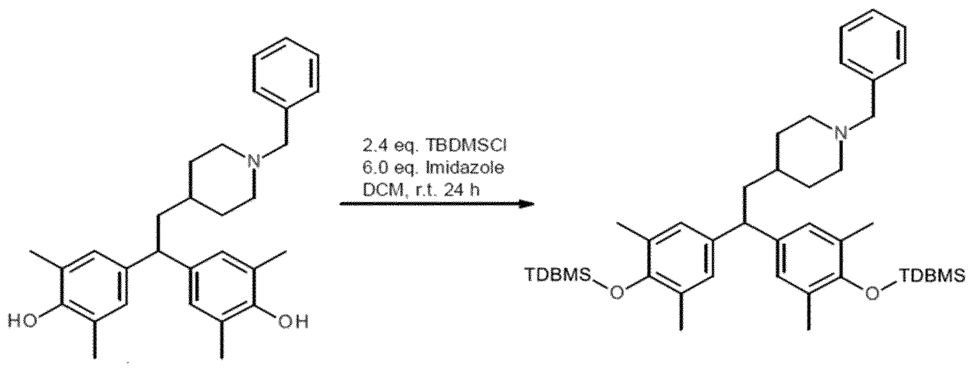


Intermediate 4

Intermediate 5

Reaction scheme 6 (step 1)

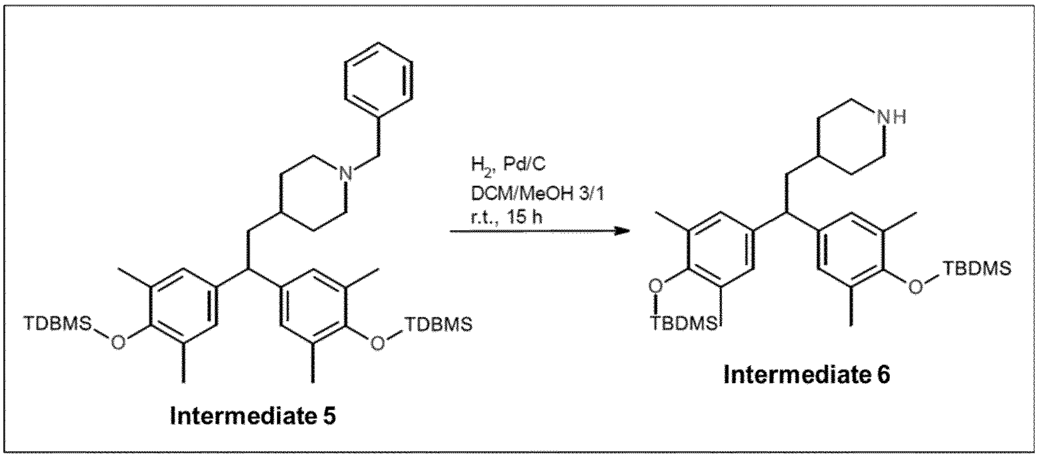


Reaction scheme 7 (step 2)

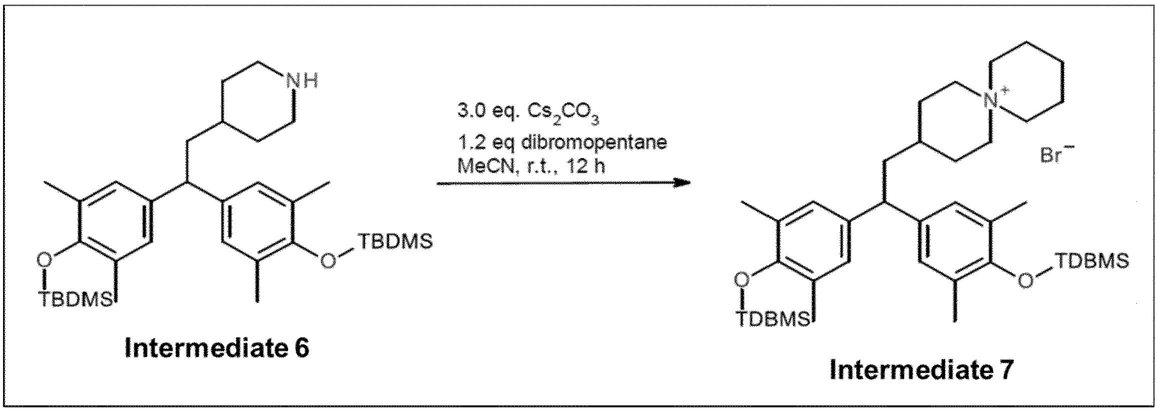


Reaction scheme 8 (step 3)

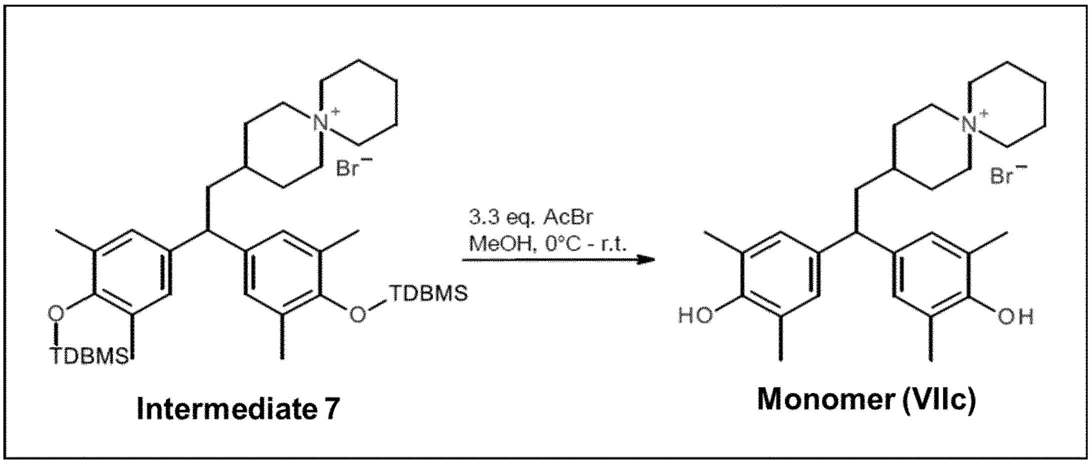


Reaction scheme 9 (step 4)

In a three-necked round-bottom flask (1000 mL) equipped with thermometer, dropping funnel, mechanical stirrer, ice bath and $N_2$ atmosphere 20 g (45.1 mmol) of Intermediate 4 was dissolved in 451 mL of DCM and 18.4 g (270.5 mmol) of Imidazole was added. The mixture was carefully evacuated & refilled with $N_2$ (three times). Reaction mixture was cooled with ice bath and 16.31 g (108.2 mmol) of tert-Butyldimethylchlorosilane (TBDMSCl) in 20 mL of DCM added to the reaction mixture dropwise. After addition, cooling was removed, and reaction mixture was stirred at room temperature overnight leading to formation of white precipitate. The reaction mixture was quenched with 200 mL of saturated $NH_4Cl$ solution and 200 mL of water were added following by extraction with 600 mL of DCM. The water phase was washed with

300 mL of DCM and the combined organic layers were washed two times with 400 mL of water and with 400 mL of brine, dried over $MgSO_4$, filtered and the solvent was removed under reduced pressure. Crude product was purified by normal phase column chromatography to give 24.92 g (65 % yield) of Intermediate 5.

In a three-necked round-bottom flask (1000 mL) equipped with mechanical stirrer, $N_2$ atmosphere and $H_2$ atmosphere 24.7 g (36.7 mmol) of Intermediate 5 was dissolved in 150 mL DCM/Methanol (3/1) under $N_2$. To that mixture 4.45 g Pd/C (10 %, 50 % wet) was added and the atmosphere was changed to $H_2$ (three times). The reaction mixture was stirred under $H_2$ atmosphere overnight at room temperature.

After 24 hours additional 3 g Pd/C (10 %, 50 % wet) was added to the reaction mixture and reaction was stirred under $H_2$ for additional 24 hours at room temperature. Crude product was two times filtered via Celite and washed with DCM (100 mL). Filtrate was evaporated giving 21 g (69 % crude yield) of Intermediate 6.

In a three-necked round-bottom flask (1 L) equipped with thermometer, mechanical stirrer, ice bath and $N_2$ atmosphere 21 g (36.1 mmol) of Intermediate 6 was suspended in 500 mL acetonitrile and 35.3 g (108.2 mmol) $Cs_2CO_3$ was added in one portion. The reaction mixture was cooled to 0°C and 10 g (43.3 mmol) 1,5-dibromopentane was added at once. The reaction mixture was stirred at 0°C for 2 hours and then stirred at room temperature overnight. After total 24 hours of reaction at room temperature additional 3 g 1,5-dibromopentane and 5 g $Cs_2CO_3$ was added to the reaction mixture. Additionally, the reaction mixture was evacuated with $N_2$ (three times) and was stirred at room temperature for additional 6 hours. The reaction mixture was filtered off via paper filter by gravity and reaction mixture was evaporated followed by precipitation in $Et_2O$, filtering and drying under high vacuum to give 18.17 g (69 % yield) of Intermediate 7.

In a three-necked round-bottom flask (1 L) equipped with thermometer, dropping funnel, mechanical stirrer, ice bath and $N_2$ atmosphere 186 mL of methanol were placed and 5.7 mL of acetyl bromide was dropwise added at 0°C. To that mixture 17 g (23.25 mmol) of Intermediate 7 in 50 mL of methanol was dropwise added at 0°C. The reaction mixture was stirred at room temperature overnight. After total 24 hours of reaction the reaction mixture was evaporated followed by precipitation in $Et_2O$, filtering and drying under high vacuum to give 12.35 g of crude product. Purification was performed using reverse phase column chromatography under neutral conditions to give 5.51 g (44 % yield) of spiro containing monomer (VIIc). Chemical structure of monomer (VIIc) was confirmed by 1H-NMR; 1H-NMR spectrum is given in Figure 7. DMSO-d6 was used as solvent.

**Example 16: Synthesis of spiro containing polymer (part of invention)**

Synthesis was performed in a 250 mL three-necked flask with oil bath, mechanical stirrer, a packed column with distillation head cooler with adjustable return ratio and condensate removal. At the beginning of synthesis 5.02 g (0.01 mol) of spiro containing monomer (VIIc) from Example 15, 2.18 g (0.01 mol) of 4,4'-Difluorobenzophenon (VIa), 70 mL of N,N-Dimethylformamide and 3.04 g (0.022 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 5 mL of N,N-Dimethylformamide were added to the reaction mixture and temperature of the reaction mixture was increased to 154°C. After 20 hours the heating of the oil bath was turned off, the viscous reaction product was cooled down and poured into cold water. The precipitated product was washed with hot water three times and was dried under vacuum at 40°C over 48 hours. The yield was 5.48 g (85.6 %). Chemical structure of spiro containing polymer from Example 16 was confirmed by 1H-NMR; 1H-NMR spectrum is given in Figure 8. DMSO-d6 was used as solvent.

**Example 17: Membrane casting of spiro containing polymer from Example 16 (part of invention)**

5 g of polymer from Example 16 were dissolved in 20 mL of N,N-Dimethylformamide under stirring at 60°C for one hour. The required amount of polymer solution was taken up with a syringe and applied directly through a 1 $\mu$m PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 18: Synthesis of amine containing monomer (VIId) (part of invention)**

A solution of 138.05 g (1.13 mol) of 2,6-dimethylphenol in 500 mL of acetic acid was placed in 2 L three-neck round-bottom flask equipped with thermometer, dropping funnel, mechanical stirrer and $N_2$ atmosphere, 500 mL of conc aq. HCl solution and 74.59 g (0.56 mol) of 2,2-Dimethoxy-N,N-dimethylethanamine were added dropwise. The reaction mixture was stirred at room temperature overnight and additional 250 mL of conc aq. HCl were added after 24 hours of reaction. The reaction mixture was stirred at room temperature overnight. After total 48 hours of reaction it was directly concentrated under vacuum to dryness and was mixed with 1.5 L of water and sonicated for 30 minutes. The solid product was collected by filtration and washed with water, then suspended in 500 mL of acetonitrile and concentrated under

vacuum to give 97 g of crude product. Then 40 g of it was purified by reverse phase column chromatography to give 30.8 g of pure product as HCl-salt followed by conversion of it in free amine form by suspension in 700 mL of 25 % ammonia solution and stirring at room temperature overnight. Obtained solid was collected by filtration, washed with water and directly freeze dried to give 25.03 g (63 % yield) of amine containing monomer (VIId).

Chemical structure of monomer (VIId) was confirmed by $^1$H-NMR; $^1$H-NMR spectrum is given in Figure 9. DMSO-d6 was used as solvent.

Same reaction conditions can be applied for synthesis of similar monomers with different length n of aliphatic chain, e.g. in case 3,3-Dimethoxy-N,N-dimethyl-1-propanamine will be used, than n is equal to 2.

**Example 19: Synthesis of amine containing polymer (part of invention)**

Synthesis was performed in a 500 mL three-necked flask with oil bath, mechanical stirrer, a packed column with distillation head cooler with adjustable return ratio and condensate removal. At the beginning of synthesis 9.4 g (0.03 mol) of amine containing monomer (VIId) from Example 18, 6.55 g (0.03 mol) of 4,4'-Difluorobenzophenon (VIa), 150 mL of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 13.36 g (90.6 %).

**Example 20: Quaternization of amine containing polymer from Example 19 (part of invention)**

10 g of the polymer from Example 19 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized amine containing polymer from Example 20 was confirmed by $^1$H-NMR; $^1$H-NMR spectrum is given in Figure 10. DMSO-d6 was used as solvent.

**Example 21: Membrane casting of amine containing polymer from Example 20 (part of invention)**

The solution of the quaternized polymer from Example 20 was directly used for preparation of the membrane. The required amount of polymer solution was taken up with a syringe and applied directly through a 1 $\mu$m PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 22: Synthesis of amine containing polymer (part of invention)**

Synthesis was performed in a 500 mL three-necked flask with oil bath, mechanical stirrer, a packed column with distillation head cooler with adjustable return ratio and condensate removal. At the beginning of synthesis 9.4 g (0.03 mol) of amine containing monomer (VIId) from Example 18, 7.63 g (0.03 mol) of 4,4'-Difluordiphenylsulfon (VIb), 160 mL of N,N-Dimethylacetamide and 9.12 g (0.066 mol) of finely ground $K_2CO_3$ were mixed under nitrogen atmosphere over one hour at room temperature. Afterwards the temperature of the reaction mixture was increased to 120°C and generated water was removed using the column over 4 hours. After four hours additional 10 mL of N,N-Dimethylacetamide were added to the reaction mixture and temperature of the reaction mixture was increased to 165°C. After 20 hours the heating of the oil bath was turned off, the viscous reaction product was cooled down and poured into cold water. The precipitated product was washed with hot water three times and was dried under vacuum at 40°C over 48 hours. The yield was 13.87 g (87.6 %).

**Example 23: Quaternization of amine containing polymer from Example 22 (part of invention)**

10 g of the polymer from Example 22 were dissolved in 40 mL of N,N-Dimethylacetamide under stirring at 60°C for one hour. After cooling of the polymer solution down to 30°C dropwise 2.5 mL of iodomethane were added to the polymer solution and polymer solution was stirred for 24 hours at 30°C leading to quaternization of the polymer. Chemical structure of quaternized piperidine containing polymer from Example 6 was confirmed by $^1$H-NMR; $^1$H-NMR spectrum is given in Figure 11. DMSO-d6 was used as solvent.

**Example 24: Membrane casting of amine containing polymer from Example 23 (part of invention)**

The solution of the quaternized polymer from Example 23 was directly used for preparation of the membrane. The required amount of polymer solution was taken up with a syringe and applied directly through a 1 μm PTFE filter on a glass plate preheated to 40°C. For the coating of the glass plate, an applicator with doctor blade was automatically pulled over the glass plate at a speed of 5 mm/s. The applied wet layer was pre-dried for 24 hours under $N_2$ atmosphere at room temperature and then finally dried for 6 hours at 60°C under vacuum.

**Example 25: Ion exchange of inventive and conventional membranes in hydroxide form**

The membranes prepared in Example 4 of WO 2021/013694 A1 (labeled as Membrane #1), Example 11 of present disclosure (labeled as Membrane #2), Example 17 of present disclosure (labeled as Membrane #3), membrane made of polymer prepared using a tertiary amine bisphenol monomer (MPDDP) described in CN 110294845 A under reaction conditions similar to Example 2 of present disclosure (labeled as Membrane #4) and commercially available anion exchange membrane FAA-3-50 were ion-exchanged using following procedure: six samples of each membrane (40 x 40 mm) were placed in fresh portions of 1 M KOH solution three times for 1 hour each at 60°C and subsequently in fresh portion of 1 M KOH solution for 24 hours at 60°C. Afterwards the membrane samples were rinsed off with deionized water and placed in fresh portions of the deionized water three times for 1 hour each at room temperature. Subsequently, the membrane samples were stored in a fresh portion of the deionized water overnight at room temperature.

**Example 26: Degradation of inventive and conventional membrane samples in 2 M KOH at 80°C**

All samples from Example 25 (six samples of each membrane type per bottle) were placed in 250 mL bottle made of thick PTFE material and equipped with gas tight screw cap, filled with 2 M KOH solution and placed in drying cabinet at 80°C for 2000 hours. After that samples were rinsed with deionized water and the ionic conductivity of each sample was measured.

**Example 27: Measurement of ionic conductivity (IC) of inventive and conventional membranes**

The ionic conductivity (IC) of ion-exchanged membrane samples from Example 25 and Example 26 was measured by means of impedance spectroscopy (EIS) in a conventional 4-electrode arrangement. The membrane sample was mounted in a commercial BT-112 cell (Bekk Tech LLC), so that the two outer Pt wires were placed under the sample and the two midpoint Pt wires above the sample. The BT-112 cell was mounted between two PTFE plates and filled with deionized water. The temperature of the deionized water was controlled by a water bath and deionized water was pumped permanently through the cell. The calculation of the membrane resistance ($R_{membrane}$) was carried out by fitting acquired EIS spectrum using a widely used R (RC) Randles equivalent circuit.

The ionic conductivity (σ) of the membrane sample is given by Equation (1):

$$\sigma = L / (R_{membrane} * A) \qquad (1)$$

where L is the distance between Pt wires (5 mm) and A is the area of the membrane sample between the two outer Pt wires. Each measurement was repeated for six samples per each membrane and a mean ± standard deviation was calculated. Commercially available anion exchange membrane FAA-3-50 was tested in the same way. Additionally, the IC of native membrane samples (non-degraded samples of as prepared membranes) from Example 25 was measured as reference.

Table 1 summarizes all measurement results. Normalized initial IC represents the normalized IC of native membrane samples (non-degraded samples of as prepared membranes), while remaining normalized IC represents the IC of degraded over 2000 hours samples.

**Table 1**: Experimental data obtained according to Examples 25 to 27 with membranes from Example 4 of WO 2021/013694 A1 (labeled as Membrane #1), Example 11 of present disclosure (labeled as Membrane #2), Example 17 of present disclosure (labeled as Membrane #3), membrane made of polymer prepared using a tertiary amine bisphenol monomer (MPDDP) described in CN 110294845 A under reaction conditions similar to Example 2 of present disclosure (labeled as Membrane #4) and commercially available anion exchange membrane FAA-3-50 from FUMATECH BWT GmbH.

| | Qualification | Normalized initial IC [%] | Remaining normalized IC [%] |
|---|---|---|---|
| Membrane 1 | prior art | 100 | 88.6 ± 0,1 |

(continued)

| | Qualification | Normalized initial IC [%] | Remaining normalized IC [%] |
|---|---|---|---|
| Membrane 2 | invention | 100 | 90.2 ± 0,1 |
| Membrane 3 | invention | 100 | 94.5 ± 0,1 |
| Membrane 4 | prior art | 100 | n/a* |
| FAA-3-50 | prior art | 100 | n/a** |
| *Membrane 4 disintegrated into several small pieces after 1000 hours in 2M KOH at 80°C. **FAA-3-50 membrane completely disintegrated after 120 hours in 2 M KOH at 80°C. | | | |

It can be seen from Table 1, that the membranes according to present invention show a higher remaining normalized IC value than the prior art membranes. Therefore, inventive compounds will maintain their ionic conductivity for longer period of time.

## Claims

1. Compound containing at least one unit of the formula (I)

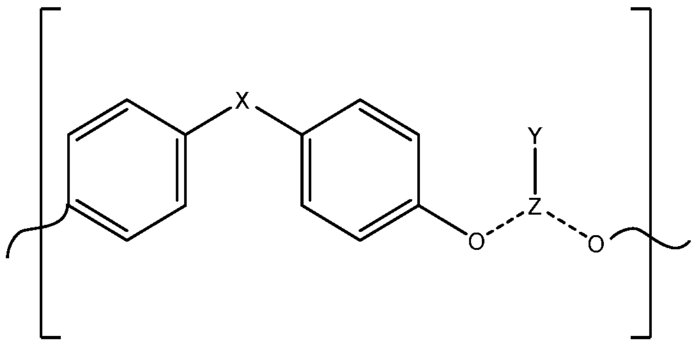

(I)

wherein X being a ketone or sulfone group,

wherein Z being a structure element comprising at least one tertiary carbon atom and at least one aromatic 6-ring directly bonded to one of the oxygen atoms,
and wherein Y being a structure element comprising at least one nitrogen atom with a positive charge and Y being bonded to said tertiary carbon atom of Z.

2. Compound according to Claim 1, **characterized in that** the compound is represented by formula (Ia) or (Ib)

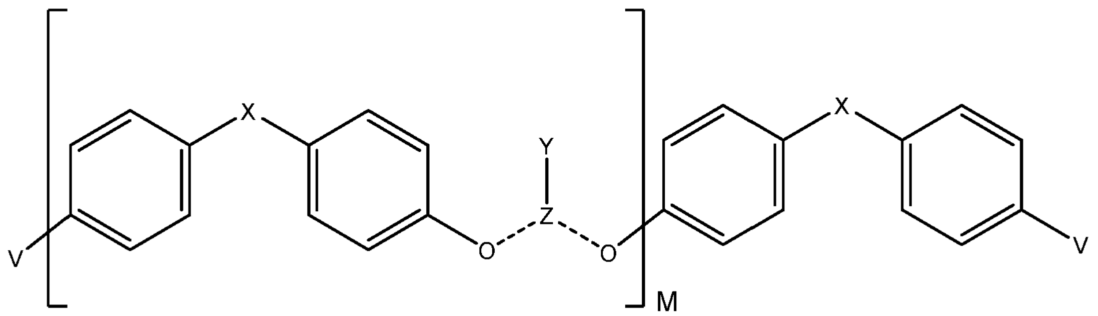

(Ia)

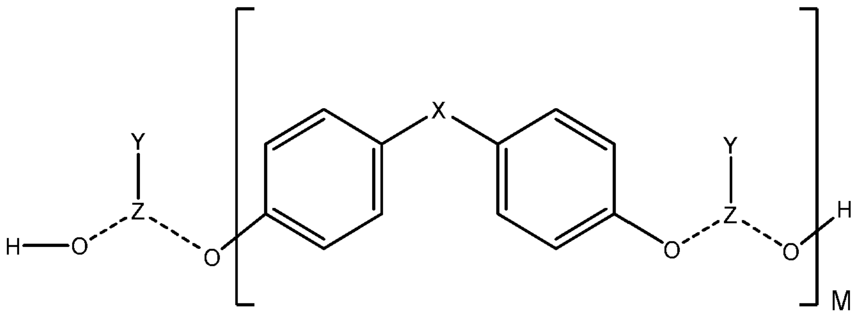
(Ib)

wherein V represents same or different halogen, preferably V being Fluor and with M being an integer from 1 to 1000, preferably M being an integer from 5 to 500.

3. Compound according to Claim 1 or 2, **characterized in that** the structure element Y represents a unit of formula (IIa), (IIb) or (IIc)

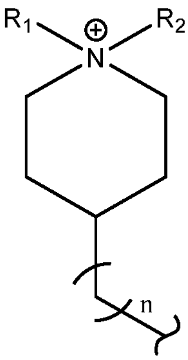
(IIa)

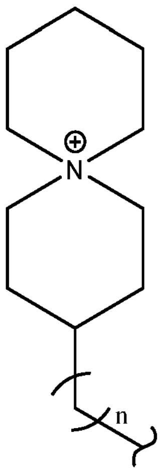
(IIb)

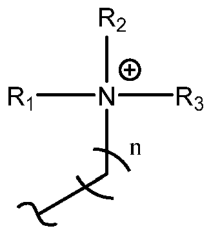
(IIc)

with n representing number of carbon atoms in the aliphatic chain and n being from 0 to 9, preferably from 0 to 5; $R_1$, $R_2$

and $R_3$ being the same or different alkyl group having from 1 to 9 carbon atoms, preferably $R_1$, $R_2$ and $R_3$ each being a methyl group.

**4.** Compound according to any one of Claims 1 to 3, **characterized in that** the structure element Y present in the compound represents in more than 5 %, preferably in more than 50 %, and most preferred in more than 90 % of its occurrence a unit of formula (IIa), (IIb) or (IIc).

**5.** Compound according to any one of Claims 1 to 4, **characterized in that** the structure element Z represents a unit of formula (III)

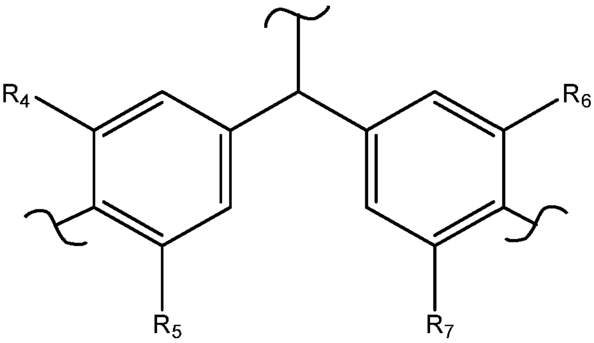

(III)

with $R_4$, $R_5$, $R_6$ and $R_7$ being the same or different alkyl group having from 1 to 4 carbon atoms, $R_4$, $R_5$, $R_6$ and $R_7$ each preferably being a methyl, iso-propyl or tert-butyl group, more preferably $R_4$, $R_5$, $R_6$ and $R_7$ each being a methyl group.

**6.** Compound according to at least one of Claims 1 to 5, **characterized in that** the compound is represented by at least one of formulas (IVa) to (IVf):

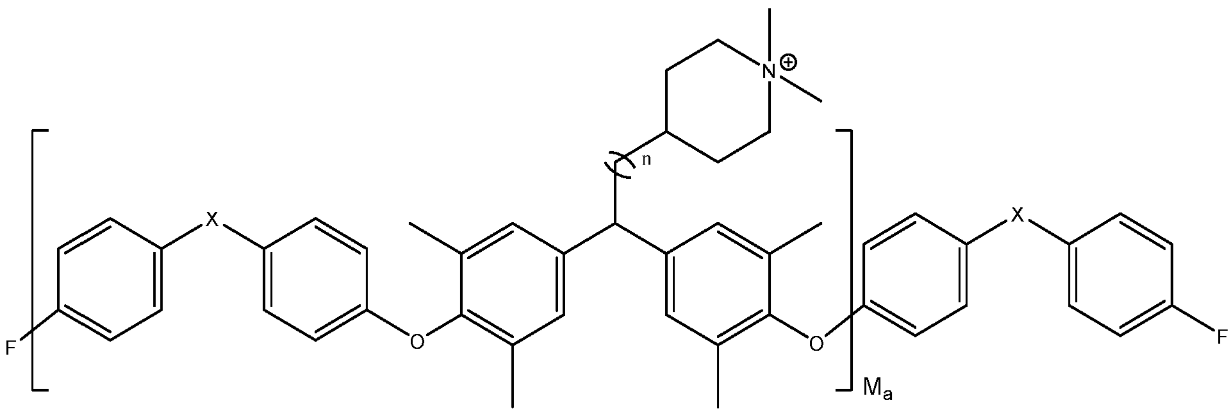

(IVa)

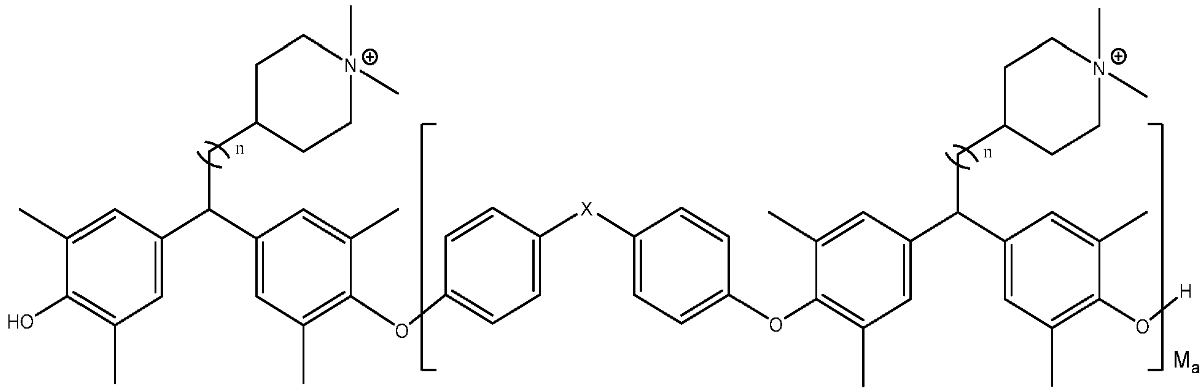

(IVb)

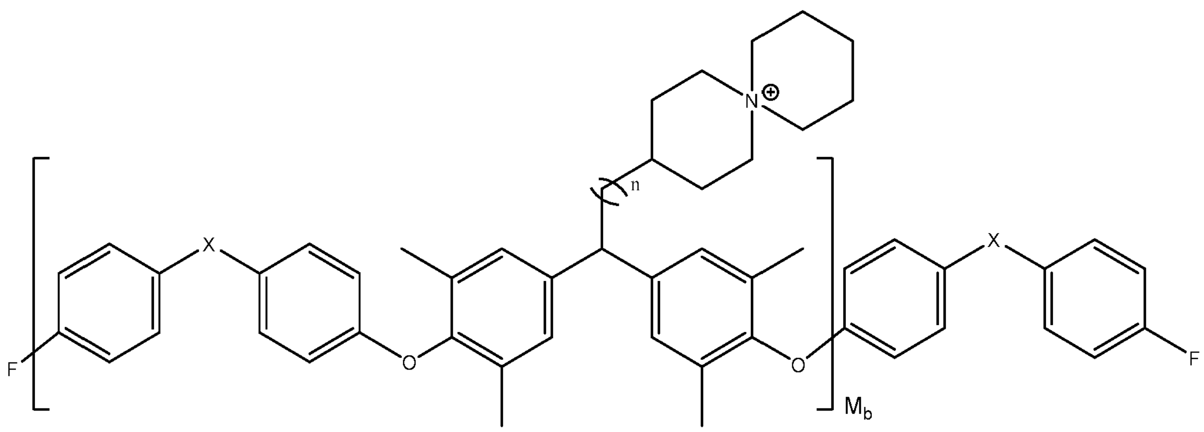

(IVc)

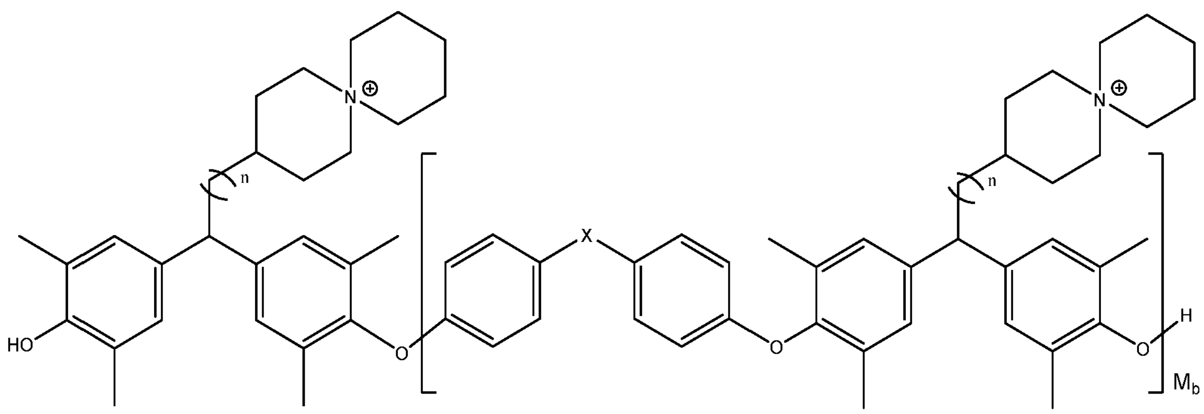

(IVd)

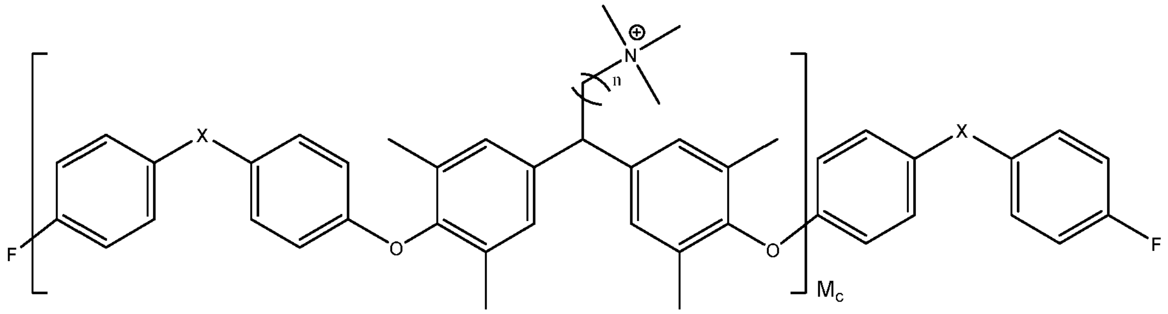

(IVe)

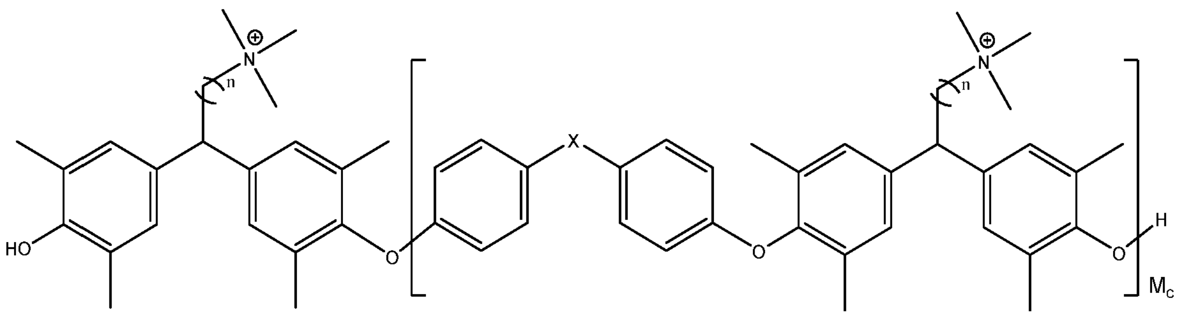

(IVf)

with n representing number of carbon atoms in the aliphatic chain and n being from 0 to 9, preferably from 0 to 5 and with $M_a$, $M_b$ and $M_c$ each being an integer of from 1 to 1000, preferably $M_a$, $M_b$, and $M_c$ each being an integer of from 5 to 500.

**7.** Compound according to any of claims 1 to 6, wherein said aromatic 6-ring directly bonded to one of the oxygen atoms is further substituted with one or more halogen and/or one or more $C_1$- to $C_4$- alkyl radicals, whereby alkyl radicals selected from the list consisting of methyl, iso-propyl, and tert-butyl are preferred ones while methyl is the most preferred alkyl.

**8.** Compound according to any of claims 1 to 6, wherein said aromatic 6-ring directly bonded to one of the oxygen atoms is free of any further substitution with one or more halogen and/or one or more $C_1$- to $C_4$- alkyl radicals.

**9.** Process for preparing compounds according to any one of Claims 1 to 8, comprising the following steps:

a) providing a first educt, wherein said first educt comprises at least one compound of the formula (VIa) or (VIb), where V represents same or different halogen, preferred Fluor,

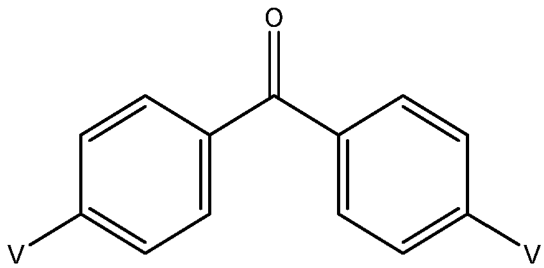
(VIa)

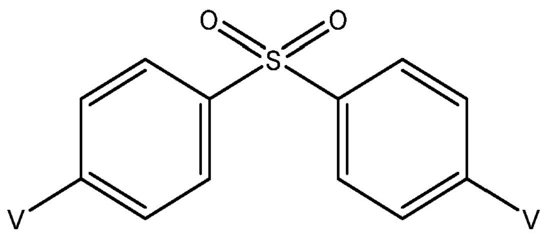
(VIb)

b) providing a second educt, wherein said second educt comprises at least one compound selected from formulas (VIIa), (VIIb), (VIIc) or (VIId)

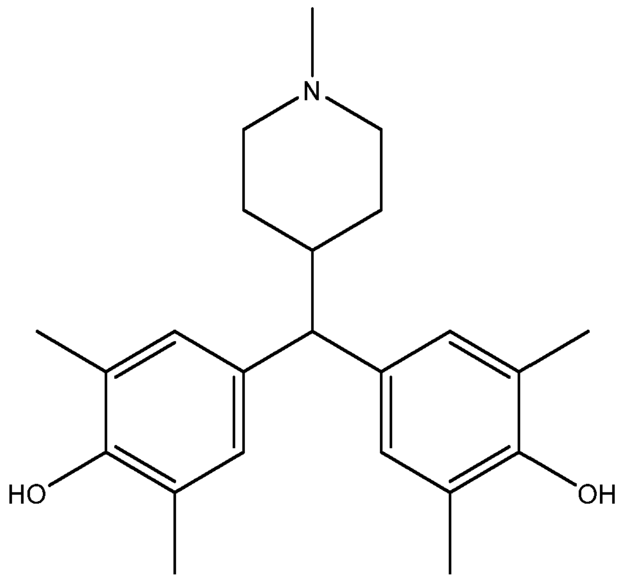

(VIIa)

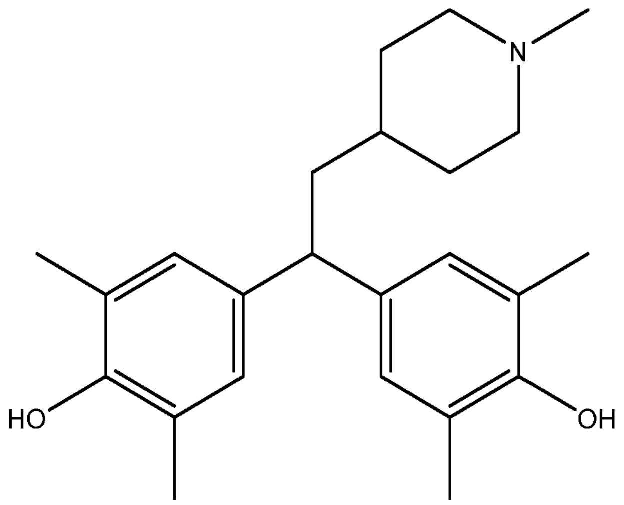

(VIIb)

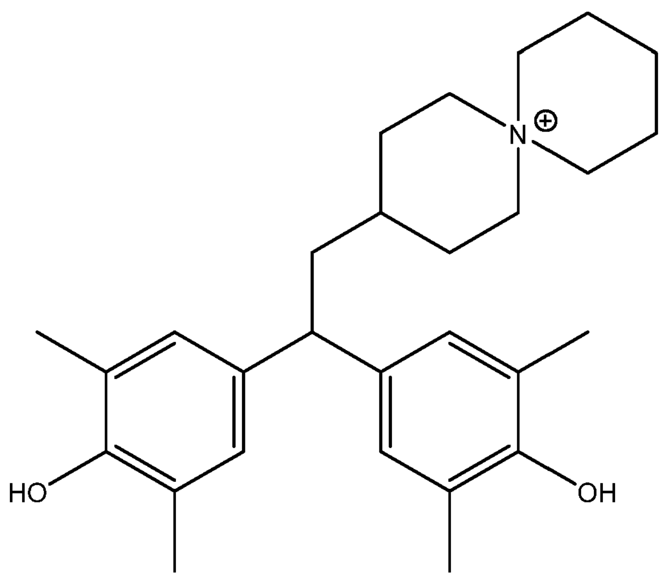

(VIIc)

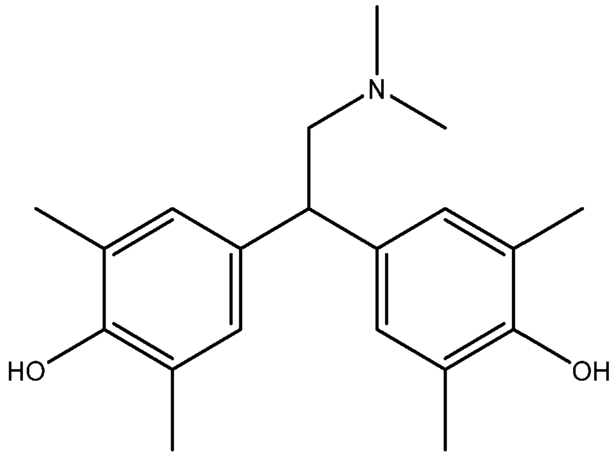

(VIId)

c) Reacting said first educt with said second educt;
d) Obtaining at least one compound according claims 1 to 8.

**10.** Process according to Claim 9, **characterized in that** an alkylating reagent, preferably a methylating reagent, is used in at least one of its steps.

**11.** Process according to Claim 9 or 10 for preparing a compound according to claim 7, wherein at least one compound of formula (VIa) or (VIb) or (VIIa) or (VIIb) or (VIIc) or (VIId) is provided as first educt or second educt respectively, **characterized in that** the aromatic rings of this compound are further substituted with one or more halogen and/or one or more $C_1$- to $C_4$-alkyl radicals, whereby alkyl radicals selected from the list consisting of methyl, iso-propyl, and tert-butyl are preferred ones while methyl is the most preferred alkyl.

**12.** Process according to Claim 9 or 10 for preparing a compound according to claim 8, wherein at least one compound of formula (VIa) or (VIb) or (VIIa) or (VIIb) or (VIIc) or (VIId) is provided as first educt or second educt respectively, **characterized in that** the aromatic rings of this compound are free of any further substitution with one or more halogen and/or one or more $C_1$- to $C_4$- alkyl radicals.

**13.** Use of a compound according to any of Claims 1 to 8 as an anion-conducting membrane or for the production of an anion-conducting membrane.

**14.** Electrochemical cell having an anion-conducting membrane, **characterized in that** said anion-conducting membrane comprises at least one compound according to at least one of claims 1 to 8.

**15.** Electrochemical cell according to claim 14, **characterized in that** electrochemical cell is a component of an electrolyzer or of a fuel cell or of a redox flow battery.

**16.** Electrochemical process performed by means of an electrochemical cell according to claim 14 or 15.

**17.** Electrochemical process performed according to claim 16, wherein said electrochemical process is an electrolysis or an electrodialysis or an electrochemical process taking place during operation of a fuel cell or an electrochemical process taking place during operation of a redox flow battery.

## Patentansprüche

**1.** Verbindung, enthaltend mindestens einen Baustein der Formel (I)

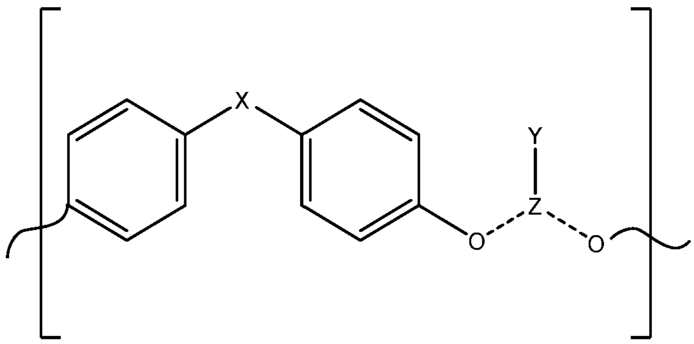

(I)

wobei X eine Keton- oder Sulfongruppe ist,
wobei Z ein Strukturelement ist, das mindestens ein tertiäres Kohlenstoffatom und mindestens einen direkt an eines der Sauerstoffatome gebundenen aromatischen 6-Ring umfasst,
und wobei Y ein Strukturelement ist, das mindestens ein Stickstoffatom mit einer positiven Ladung umfasst, wobei Y an das tertiäre Kohlenstoffatom von Z gebunden ist.

2. Verbindung nach Anspruch 1, **dadurch gekennzeichnet, dass** die Verbindung durch Formel (Ia) oder (Ib) wiedergegeben wird:

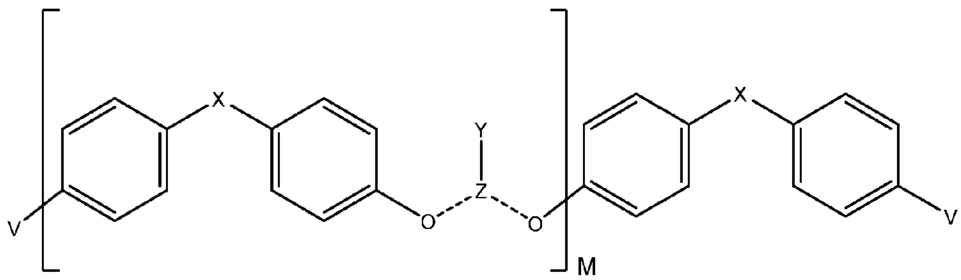

(Ia)

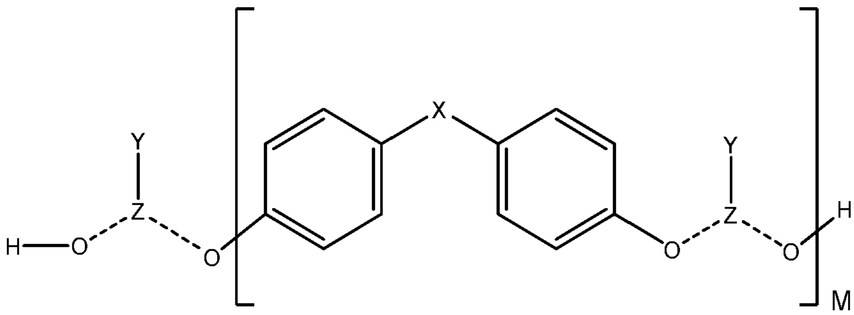

(Ib)

wobei V für gleiches oder verschiedenes Halogen steht, wobei V vorzugsweise F ist, und wobei M eine ganze Zahl von 1 bis 1000 ist, wobei M vorzugsweise eine ganze Zahl von 5 bis 500 ist.

3. Verbindung nach Anspruch 1 oder 2, **dadurch gekennzeichnet, dass** das Strukturelement Y für einen Baustein der Formel (IIa), (IIb) oder (IIc) steht:

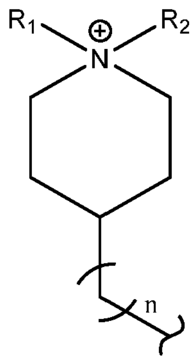

(IIa)

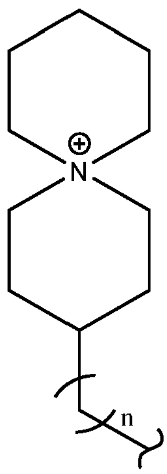

(IIb)

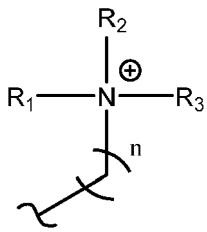

(IIc)

wobei n für die Zahl der Kohlenstoffatome in der aliphatischen Kette steht und n gleich 0 bis 9, vorzugsweise 0 bis 5, ist; $R_1$, $R_2$ und $R_3$ gleiche oder verschiedene Alkylgruppen mit 1 bis 9 Kohlenstoffatomen sind und vorzugsweise $R_1$, $R_2$ und $R_3$ jeweils eine Methylgruppe sind.

**4.** Verbindung nach einem der Ansprüche 1 bis 3, **dadurch gekennzeichnet, dass** das in der Verbindung vorliegende Strukturelement Y bei mehr als 5 %, vorzugsweise bei mehr als 50 % und ganz besonders bevorzugt bei mehr als 90 % seines Auftretens ein Baustein der Formel (IIa), (IIb) oder (IIc) ist.

**5.** Verbindung nach einem der Ansprüche 1 bis 4, **dadurch gekennzeichnet, dass** das Strukturelement Z für einen Baustein der Formel (III) steht:

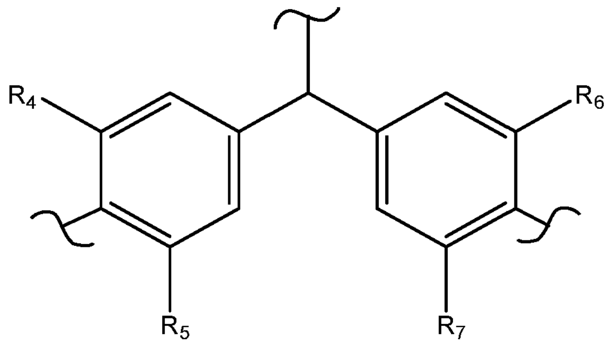

(III)

wobei $R_4$, $R_5$, $R_6$ und $R_7$ gleiche oder verschiedene Alkylgruppen mit 1 bis 4 Kohlenstoffatomen sind, wobei $R_4$, $R_5$, $R_6$ und $R_7$ jeweils vorzugsweise eine Methyl-, Isopropyl- oder tert-Butylgruppe sind und weiter bevorzugt $R_4$, $R_5$, $R_6$ und $R_7$ jeweils eine Methylgruppe sind.

6. Verbindung nach mindestens einem der Ansprüche 1 bis 5, **dadurch gekennzeichnet, dass** die Verbindung durch mindestens eine der Formeln (IVa) bis (IVf) wiedergegeben wird:

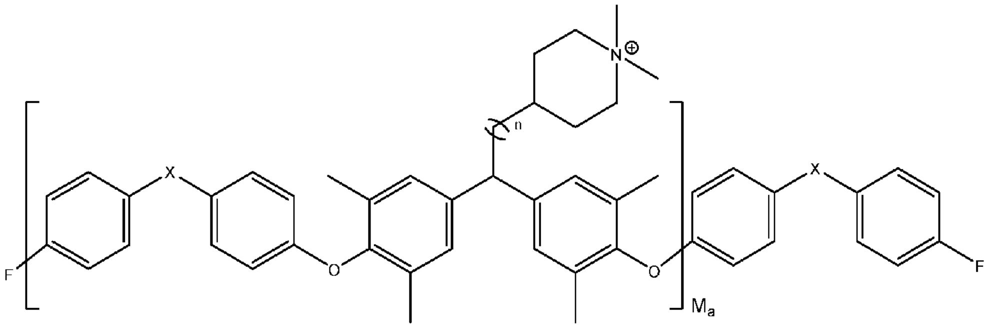

(IVa)

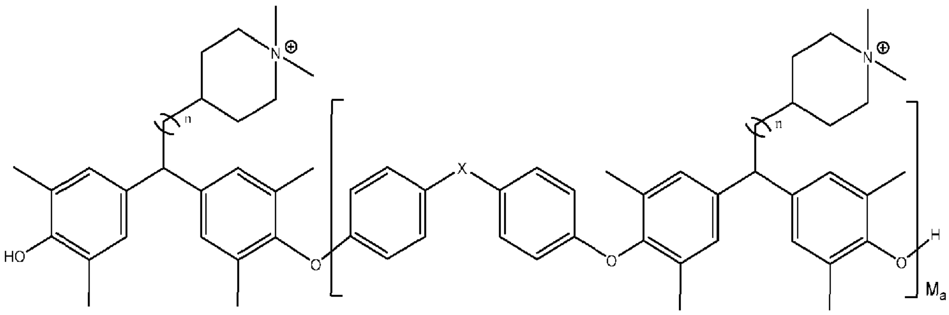

(IVb)

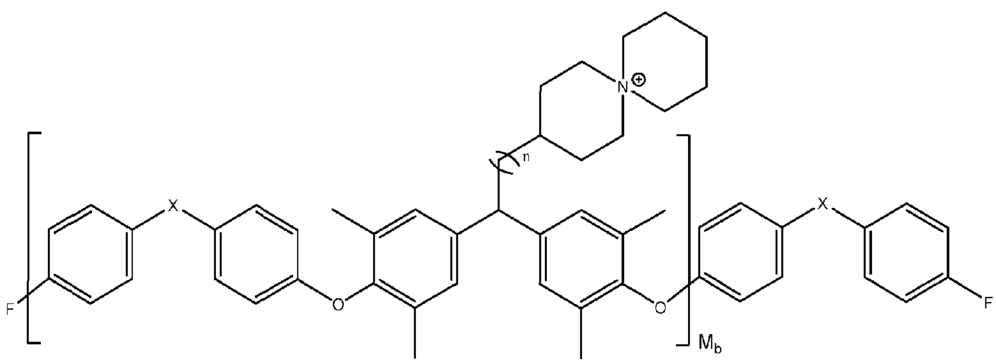

(IVc)

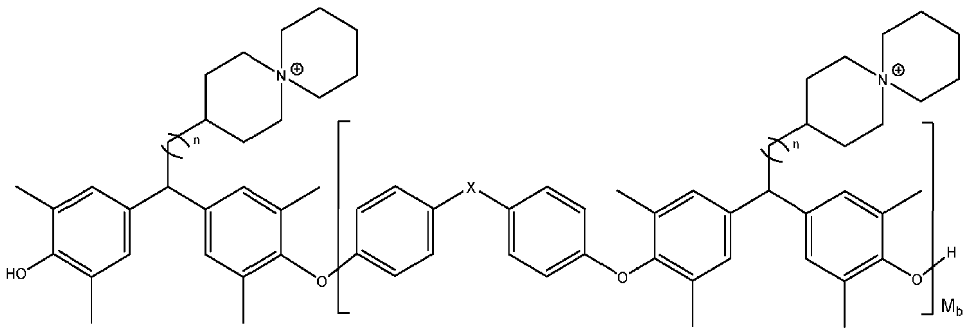

(IVd)

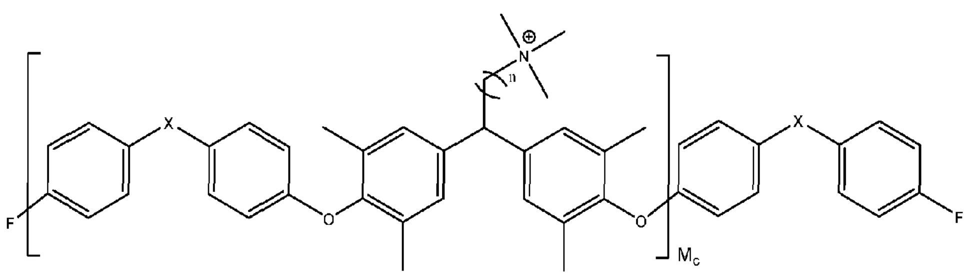

(IVe)

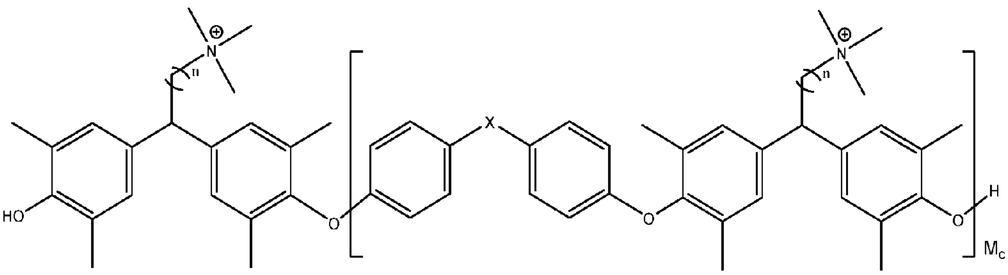

(IVf)

wobei n für die Zahl der Kohlenstoffatome in der aliphatischen Kette steht und n gleich 0 bis 9, vorzugsweise 0 bis 5, ist und wobei $M_a$, $M_b$ und $M_c$ jeweils eine ganze Zahl von 1 bis 1000 sind, vorzugsweise $M_a$, $M_b$ und $M_c$ jeweils eine ganze Zahl von 5 bis 500 sind.

**7.** Verbindung nach einem der Ansprüche 1 bis 6, wobei der direkt an eines der Sauerstoffatome gebundene aromatische 6-Ring ferner durch ein oder mehrere Halogene und/oder einen oder mehrere $C_1$- bis $C_4$-Alkylreste substituiert ist, wobei Alkylreste ausgewählt aus der Liste bestehend aus Methyl, Isopropyl und tert-Butyl bevorzugt sind, während Methyl als Alkyl am meisten bevorzugt ist.

**8.** Verbindung nach einem der Ansprüche 1 bis 6, wobei der direkt an eines der Sauerstoffatome gebundene aromatische 6-Ring frei von jeglicher weiterer Substitution durch ein oder mehrere Halogene und/oder einen oder mehrere $C_1$- bis $C_4$-Alkylreste ist.

**9.** Verfahren zur Herstellung von Verbindungen nach einem der Ansprüche 1 bis 8, das die folgenden Schritte umfasst:

a) Bereitstellen eines ersten Edukts, wobei das erste Edukt mindestens eine Verbindung der Formel (VIa) oder (VIb) umfasst, wobei V für gleiches oder verschiedenes Halogen, bevorzugt Fluor, steht,

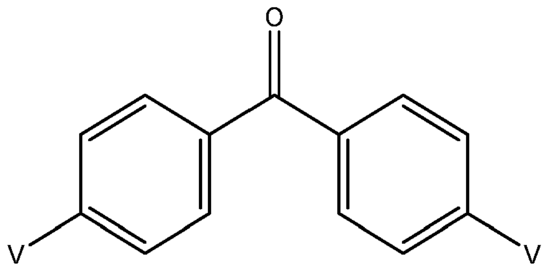

(VIa)

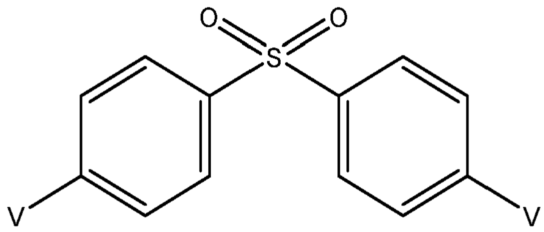

(VIb)

b) Bereitstellen eines zweiten Edukts, wobei das zweite Edukt mindestens eine Verbindung umfasst, die aus Verbindungen der Formeln (VIIa), (VIIb), (VIIc) oder (VIId) ausgewählt ist,

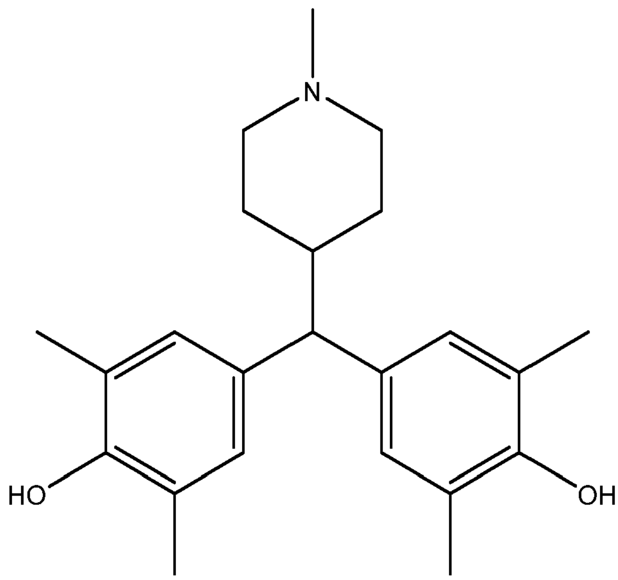

(VIIa)

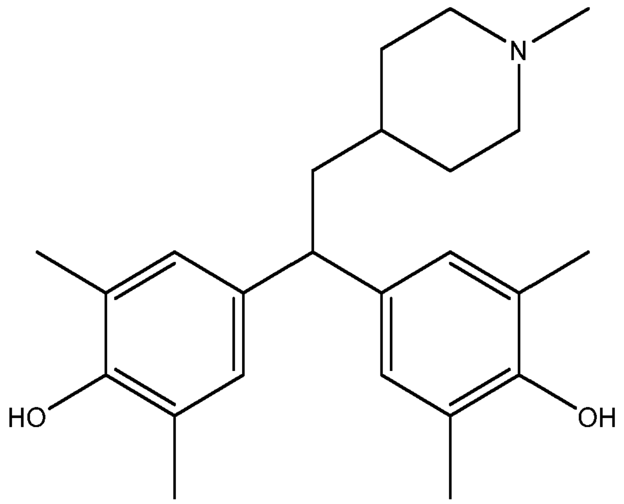

(VIIb)

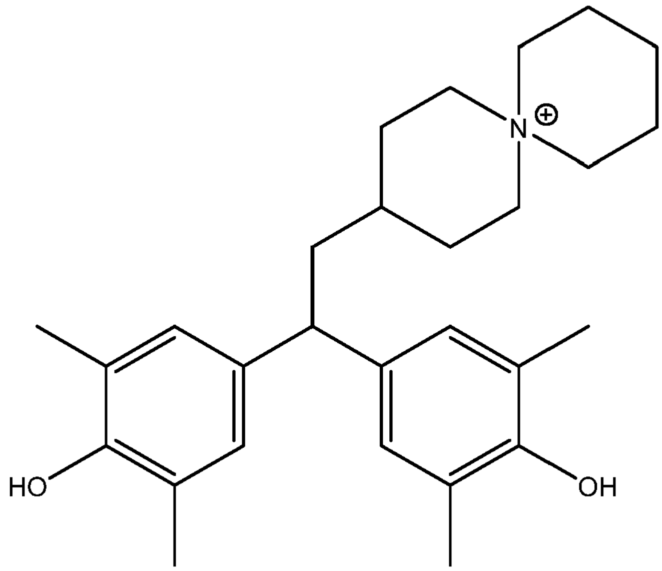

(VIIc)

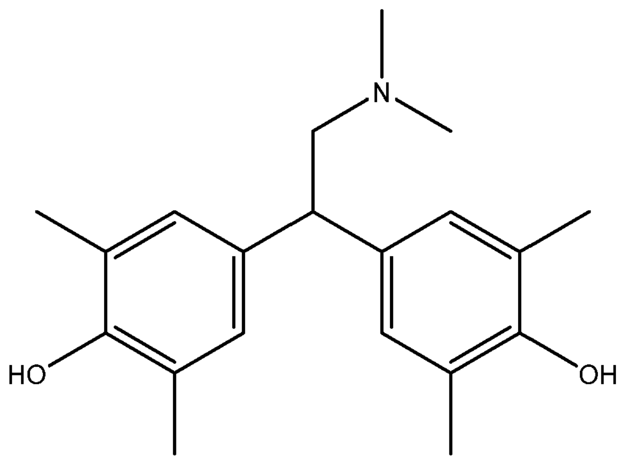

(VIId)

c) Umsetzen des ersten Edukts mit dem zweiten Edukt;
d) Erhalten mindestens einer Verbindung nach den Ansprüchen 1 bis 8.

**10.** Verfahren nach Anspruch 9, **dadurch gekennzeichnet, dass** sie mindestens einem seiner Schritte ein Alkylierungsreagens, vorzugsweise ein Methylierungsreagens, verwendet wird.

11. Verfahren nach Anspruch 9 oder 10 zur Herstellung einer Verbindung nach Anspruch 7, wobei mindestens eine Verbindung der Formel (VIa) oder (VIb) oder (VIIa) oder (VIIb) oder (VIIc) oder (VIId) als erstes Edukt bzw. zweites Edukt bereitgestellt wird, **dadurch gekennzeichnet, dass** die aromatischen Ringe dieser Verbindung ferner durch ein oder mehrere Halogen und/oder einen oder mehrere $C_1$- bis $C_4$-Alkylreste substituiert sind, wobei Alkylreste ausgewählt aus der Liste bestehend aus Methyl, Isopropyl und tert-Butyl bevorzugt sind, während Methyl als Alkyl am meisten bevorzugt ist.

12. Verfahren nach Anspruch 9 oder 10 zur Herstellung einer Verbindung nach Anspruch 8, wobei mindestens eine Verbindung der Formel (VIa) oder (VIb) oder (VIIa) oder (VIIb) oder (VIIc) oder (VIId) als erstes Edukt bzw. zweites Edukt bereitgestellt wird, **dadurch gekennzeichnet, dass** die aromatischen Ringe dieser Verbindung frei von jeglicher weiterer Substitution durch ein oder mehrere Halogene und/oder einen oder mehrere $C_1$-bis $C_4$-Alkylreste ist.

13. Verwendung einer Verbindung nach einem der Ansprüche 1 bis 8 als Anionen leitende Membran oder zur Herstellung einer Anionen leitenden Membran.

14. Elektrochemische Zelle mit einer Anionen leitenden Membran, **dadurch gekennzeichnet, dass** die Anionen leitende Membran mindestens eine Verbindung nach mindestens einem der Ansprüche 1 bis 8 umfasst.

15. Elektrochemische Zelle nach Anspruch 14, **dadurch gekennzeichnet, dass** es sich bei der elektrochemischen Zelle um ein Bauteil eines Elektrolyseurs oder einer Brennstoffzelle oder einer Redox-Flow-Batterie handelt.

16. Elektrochemischer Prozess, durchgeführt mit Hilfe einer elektrochemischen Zelle nach Anspruch 14 oder 15.

17. Elektrochemischer Prozess, durchgeführt nach Anspruch 16, wobei es sich bei dem elektrochemischen Prozess um eine Elektrolyse oder eine Elektrodialyse oder einen elektrochemischen Prozess, der während des Betriebs einer Brennstoffzelle stattfindet, oder einen elektrochemischen Prozess, der während des Betriebs einer Redox-Flow-Batterie stattfindet, handelt.

## Revendications

1. Composé contenant au moins une unité de la formule (I)

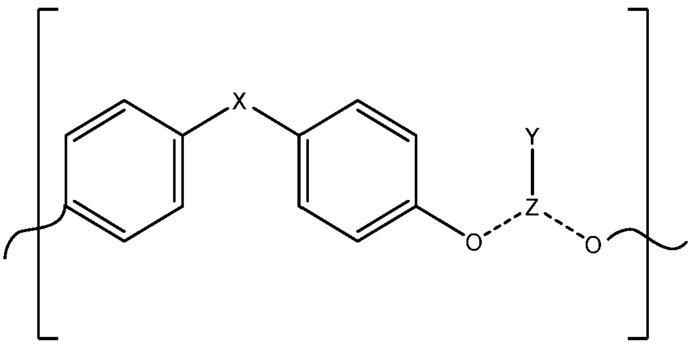

(I)

dans lequel X est un groupe cétone ou sulfone,
dans lequel Z est un élément de structure comprenant au moins un atome de carbone tertiaire et au moins un cycle aromatique à 6 chaînons directement lié à l'un des atomes d'oxygène,
et dans lequel Y est un élément de structure comprenant au moins un atome d'azote doté d'une charge positive et Y étant lié audit atome de carbone tertiaire de Z.

2. Composé selon la revendication 1, **caractérisé en ce que** le composé est représenté par la formule (Ia) ou (Ib)

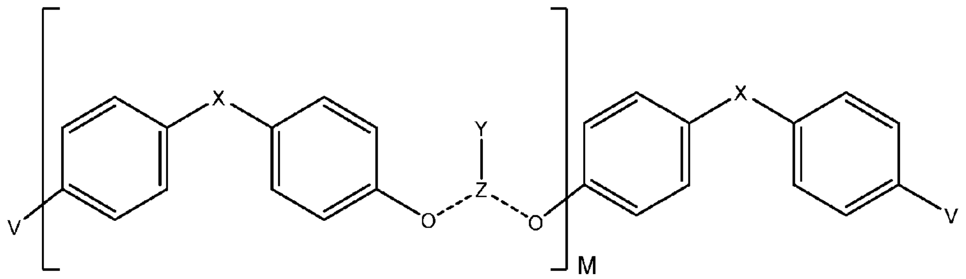

(Ia)

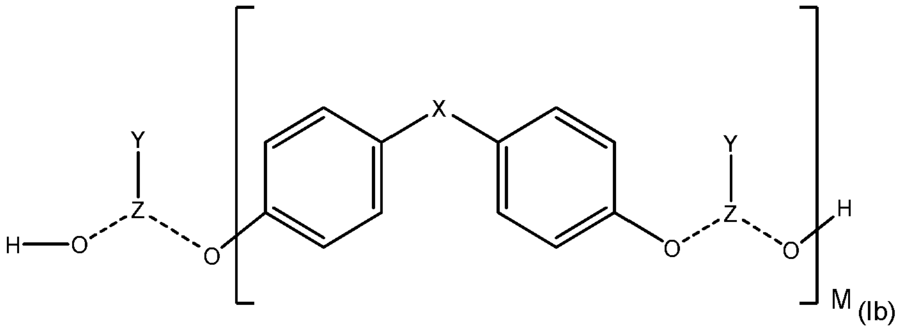

(Ib)

dans lequel V représente un halogène identique ou différent, de préférence V étant fluor et M étant un entier de 1 à 1 000, de préférence M étant un entier de 5 à 500.

3. Composé selon la revendication 1 ou 2, **caractérisé en ce que** l'élément de structure Y représente une unité de formule (IIa), (IIb) ou (IIc)

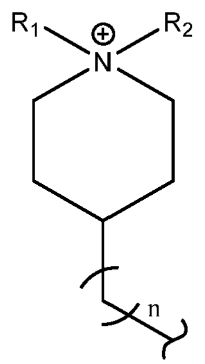

(IIa)

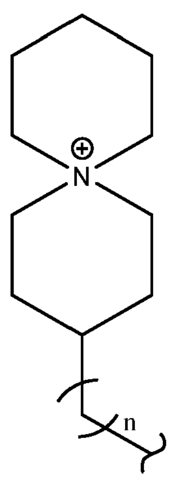

(IIb)

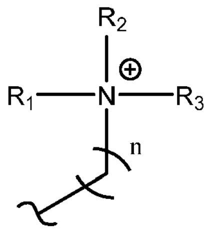

(IIc)

n représentant le nombre d'atomes de carbone dans la chaîne aliphatique et n étant de 0 à 9, de préférence de 0 à 5 ; $R_1$, $R_2$ et $R_3$ étant un groupe alkyle identique ou différent ayant de 1 à 9 atomes de carbone, de préférence $R_1$, $R_2$ et $R_3$ étant chacun un groupe méthyle.

**4.** Composé selon l'une quelconque des revendications 1 à 3, **caractérisé en ce que** l'élément de structure Y présent dans le composé représente dans plus de 5 %, de préférence dans plus de 50 %, et de la manière la plus préférée dans plus de 90 % de son occurrence, une unité de formule (IIa), (IIb) ou (IIc).

**5.** Composé selon l'une quelconque des revendications 1 à 4, **caractérisé en ce que** l'élément de structure Z représente une unité de formule (III)

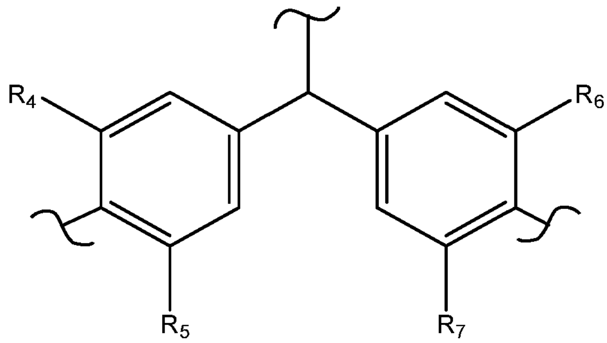

(III)

$R_4$, $R_5$, $R_6$ et $R_7$ étant un groupe alkyle identique ou différent ayant de 1 à 4 atomes de carbone, $R_4$, $R_5$, $R_6$ et $R_7$ étant chacun préférablement un groupe méthyle, isopropyle ou tert-butyle, plus préférablement $R_4$, $R_5$, $R_6$ et $R_7$ étant chacun un groupe méthyle.

**6.** Composé selon au moins l'une des revendications 1 à 5, **caractérisé en ce que** le composé est représenté par au moins une des formules (IVa) à (IVf) :

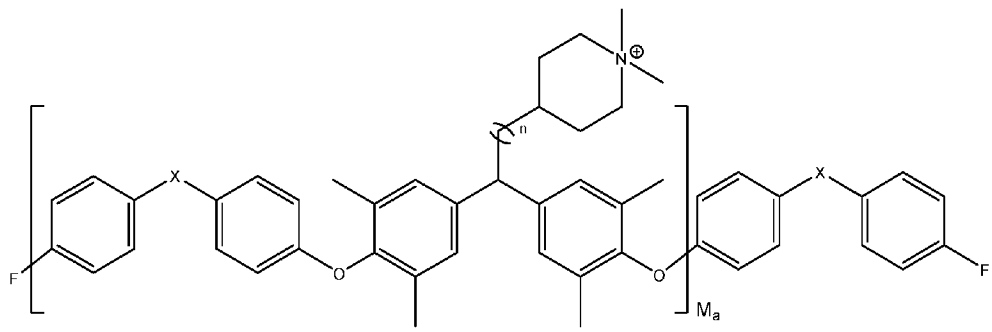

(IVa)

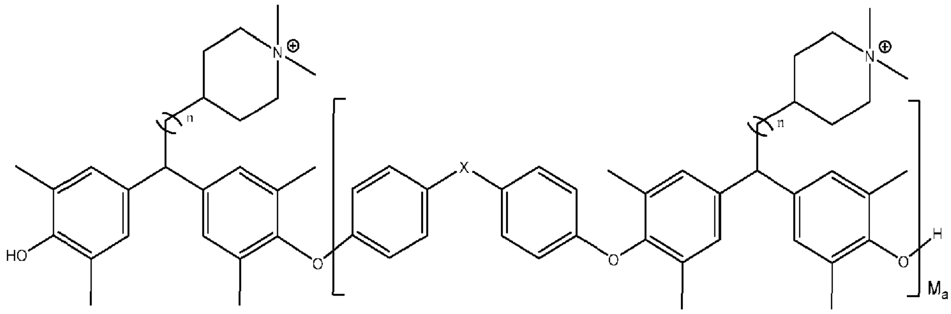

(IVb)

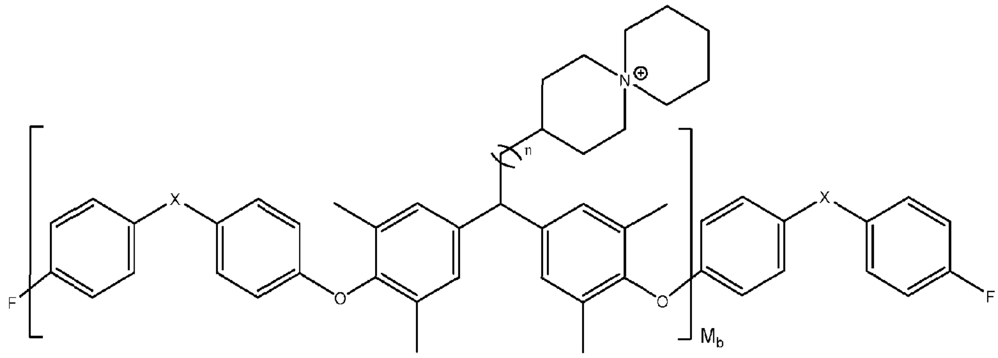

(IVc)

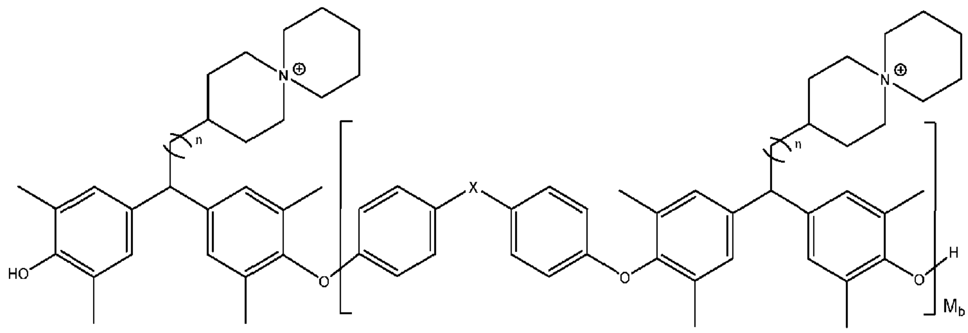

(IVd)

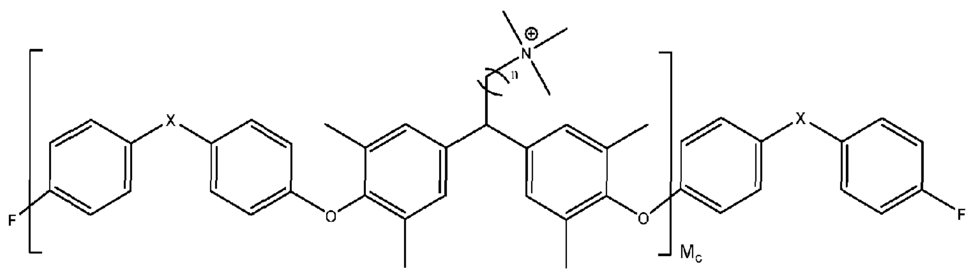

(IVe)

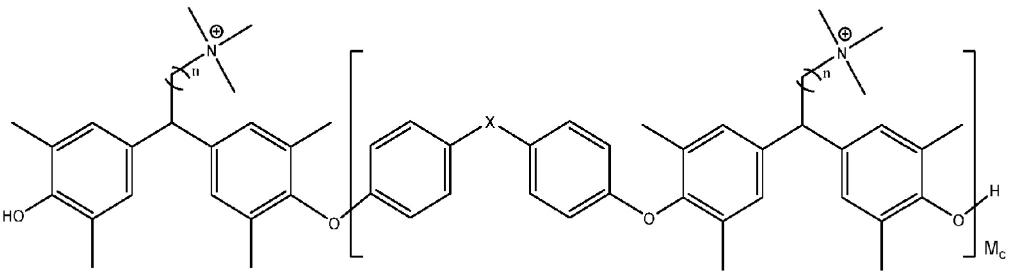

(IVf)

n représentant le nombre d'atomes de carbone dans la chaîne aliphatique et n étant de 0 à 9, de préférence de 0 à 5 et $M_a$, $M_b$ et $M_c$ étant chacun un entier de 1 à 1 000, de préférence $M_a$, $M_b$ et $M_c$ étant chacun un entier de 5 à 500.

**7.** Composé selon l'une quelconque des revendications 1 à 6, dans lequel ledit cycle aromatique à 6 chaînons directement lié à l'un des atomes d'oxygène est en outre substitué par un ou plusieurs halogènes et/ou un ou plusieurs radicaux $C_1$- à $C_4$- alkyle, moyennant quoi les radicaux alkyle choisis dans la liste constituée par méthyle, isopropyle et tert-butyle sont préférés, tandis que méthyle est l'alkyle le plus préféré.

**8.** Composé selon l'une quelconque des revendications 1 à 6, dans lequel ledit cycle aromatique à 6 chaînons directement lié à l'un des atomes d'oxygène, est exempt de toute autre substitution par un ou plusieurs halogènes et/ou un ou plusieurs radicaux $C_1$- à $C_4$- alkyle.

**9.** Procédé pour la préparation de composés selon l'une quelconque des revendications 1 à 8, comprenant les étapes suivantes :

a) fourniture d'un premier éduit, dans lequel ledit premier éduit comprend au moins un composé de formule (VIa) ou (VIb), où V représente un halogène identique ou différent, de préférence fluor,

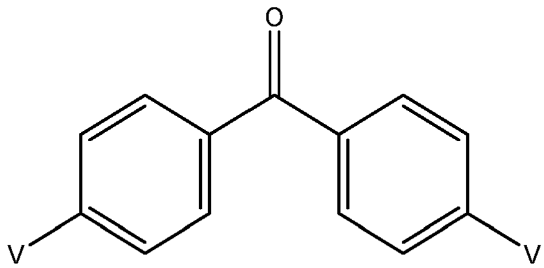

(VIa)

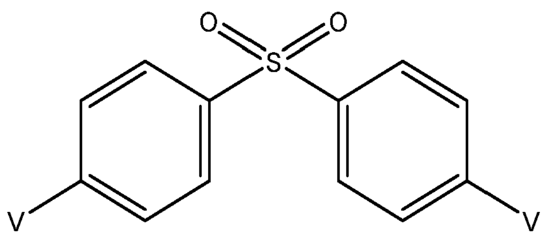

(VIb)

b) fourniture d'un deuxième éduit, dans lequel ledit deuxième éduit comprend au moins un composé choisi parmi les formules (VIIa), (VIIb), (VIIc) ou (VIId)

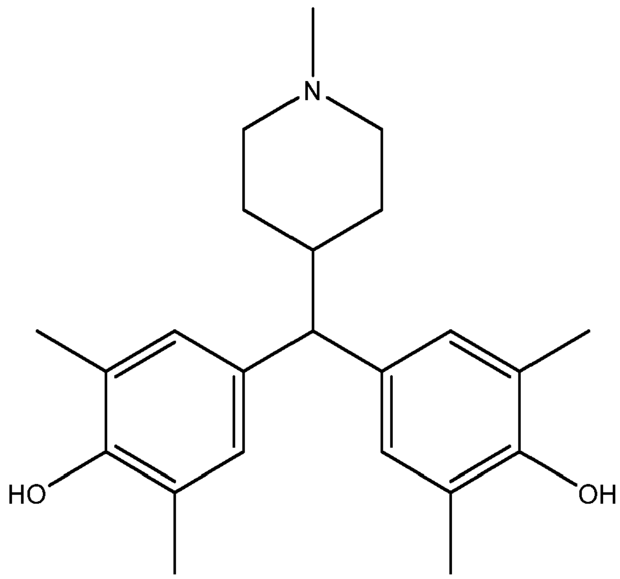

(VIIa)

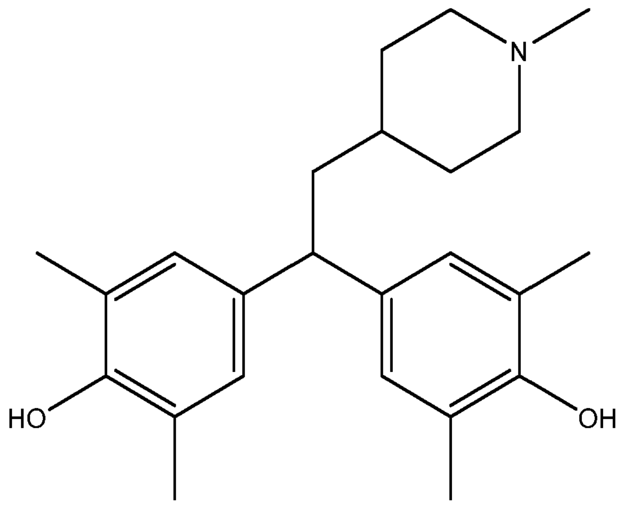

(VIIb)

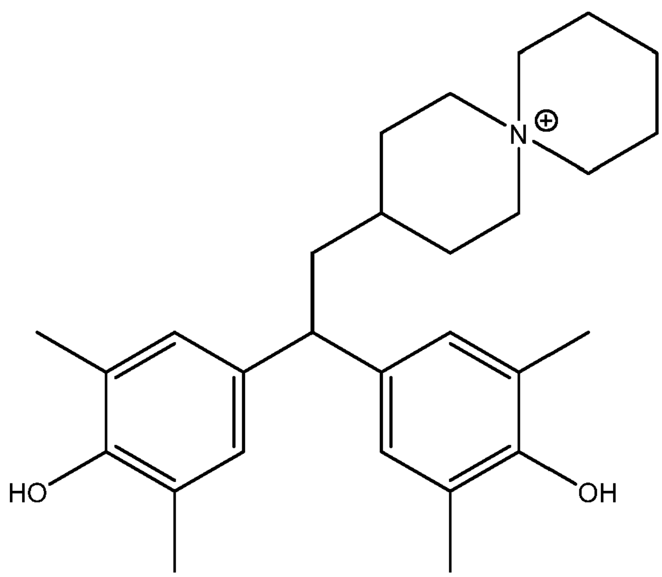

(VIIc)

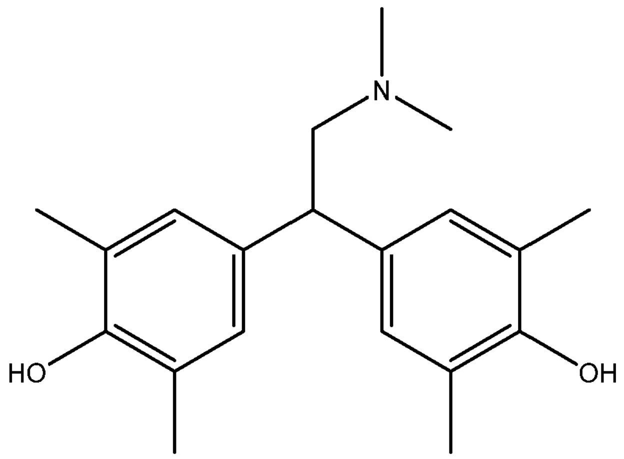

(VIId)

c) mise en réaction dudit premier éduit avec ledit deuxième éduit ;
d) obtention d'au moins un composé selon les revendications 1 à 8.

**10.** Procédé selon la revendication 9, **caractérisé en ce que** l'on utilise dans au moins une de ses étapes un réactif d'alkylation, de préférence un réactif de méthylation.

**11.** Procédé selon la revendication 9 ou 10 pour la préparation d'un composé selon la revendication 7, dans lequel au moins un composé de formule (VIa) ou (VIb) ou (VIIa) ou (VIIb) ou (VIIc) ou (VIId) est fourni en tant que premier éduit ou deuxième éduit respectivement, **caractérisé en ce que** les cycles aromatiques de ce composé sont en outre substitués par un ou plusieurs halogènes et/ou un ou plusieurs radicaux $C_1$- à $C_4$- alkyle, moyennant quoi les radicaux alkyle choisis dans la liste constituée par méthyle, isopropyle et tert-butyle sont préférés, tandis que méthyle est l'alkyle le plus préféré.

**12.** Procédé selon la revendication 9 ou 10 pour la préparation d'un composé selon la revendication 8, dans lequel au moins un composé de formule (VIa) ou (VIb) ou (VIIa) ou (VIIb) ou (VIIc) ou (VIId) est fourni en tant que premier éduit ou deuxième éduit respectivement, **caractérisé en ce que** les cycles aromatiques de ce composé sont exempts de toute autre substitution par un ou plusieurs halogènes et/ou un ou plusieurs radicaux $C_1$-à $C_4$- alkyle.

**13.** Utilisation d'un composé selon l'une quelconque des revendications 1 à 8 comme une membrane conductrice d'anions ou pour la production d'une membrane conductrice d'anions.

**14.** Cellule électrochimique ayant une membrane conductrice d'anions, **caractérisée en ce que** ladite membrane conductrice d'anions comprend au moins un composé selon au moins l'une des revendications 1 à 8.

**15.** Cellule électrochimique selon la revendication 14, **caractérisée en ce que** la cellule électrochimique est un composant d'un électrolyseur, d'une pile à combustible ou d'une batterie redox.

**16.** Procédé électrochimique réalisé au moyen d'une cellule électrochimique selon la revendication 14 ou 15.

**17.** Procédé électrochimique réalisé selon la revendication 16, dans lequel ledit procédé électrochimique est une électrolyse ou une électrodialyse ou un procédé électrochimique ayant lieu pendant le fonctionnement d'une pile à combustible ou un procédé électrochimique ayant lieu pendant le fonctionnement d'une batterie rédox.

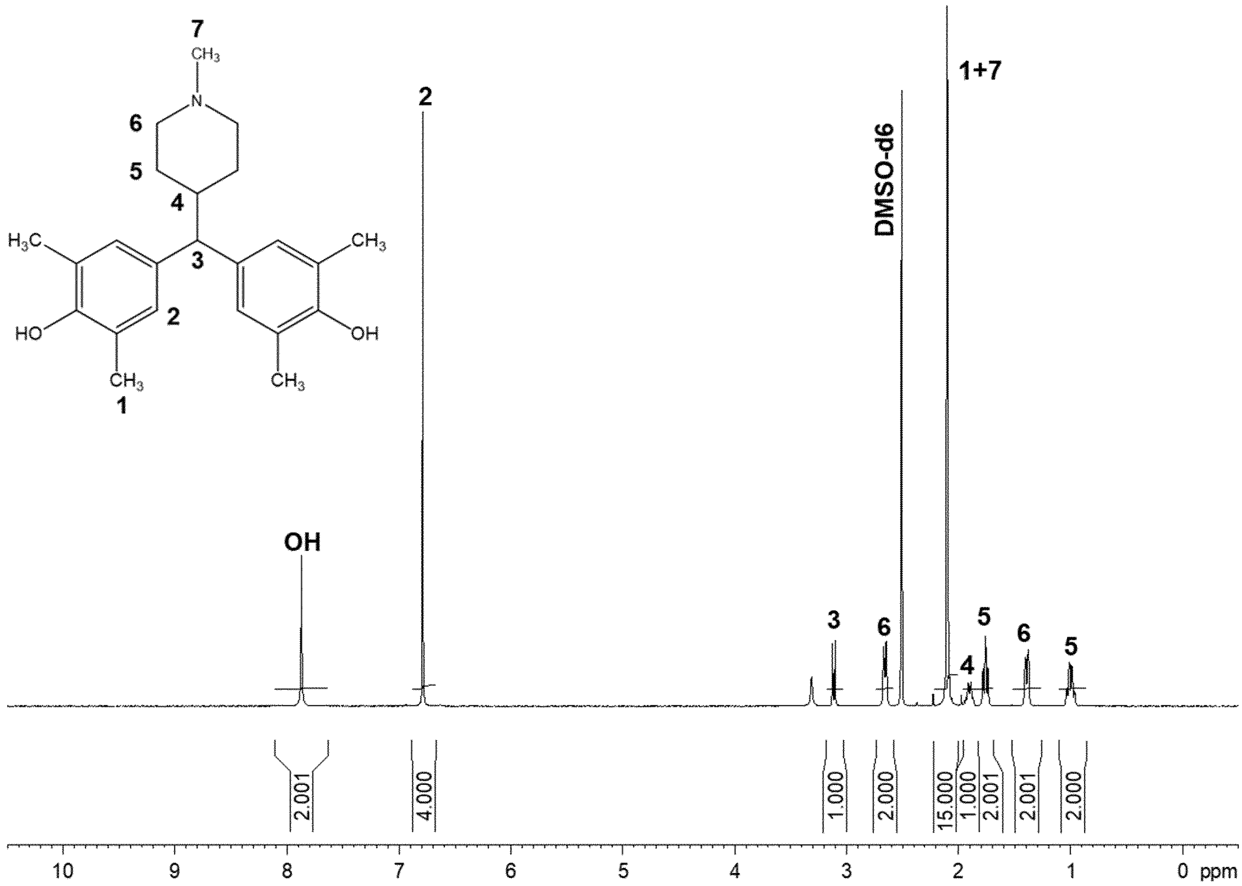
2
DMSO-d6
1+7
OH
3
6
5
4
6
5
2.001
4.000
1.000
2.000
15.000
1.000
2.001
2.001
2.000
10
9
8
7
6
5
4
3
2
1
0 ppm

Fig. 1

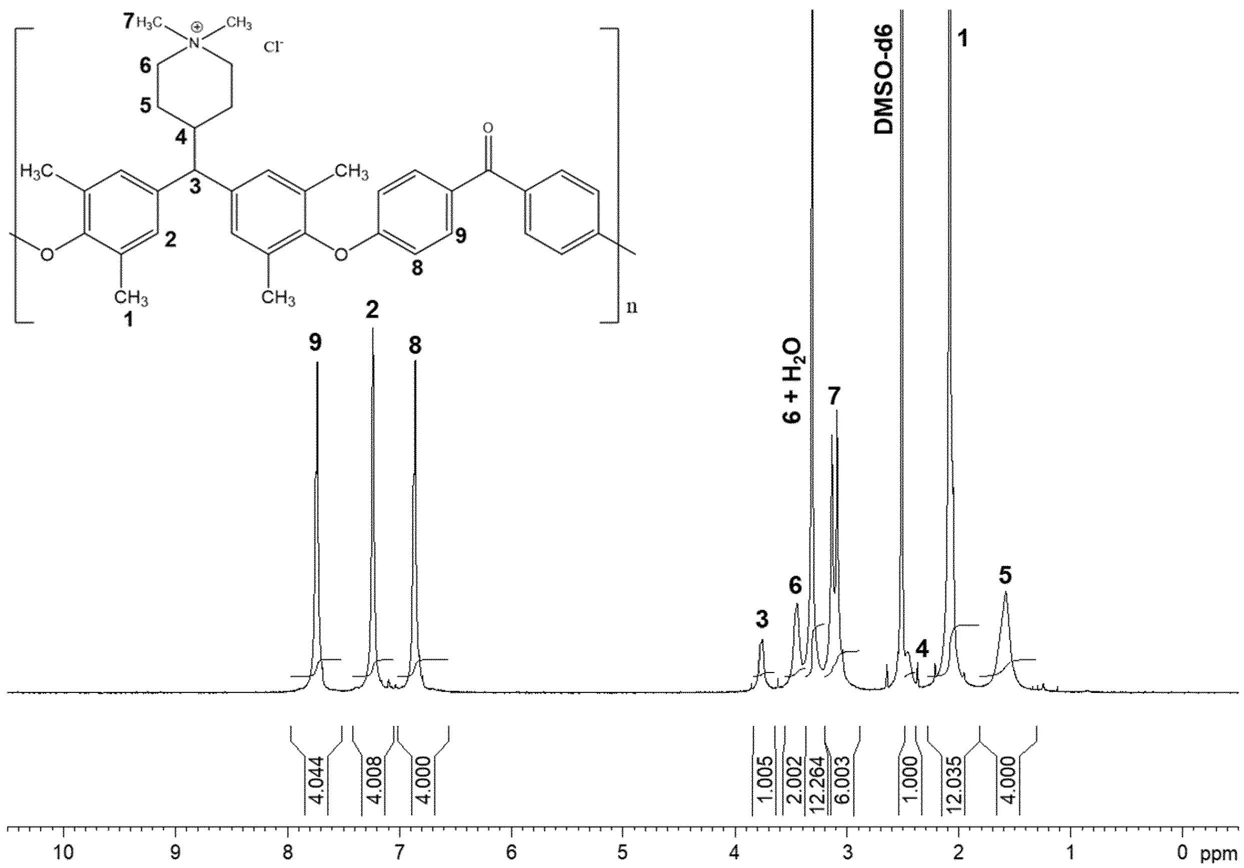
DMSO-d6
6 + H$_2$O
9
2
8
7
6
3
4
5
1
4.044
4.008
4.000
1.005
2.002
12.264
6.003
1.000
12.035
4.000
10
9
8
7
6
5
4
3
2
1
0 ppm

Fig. 2

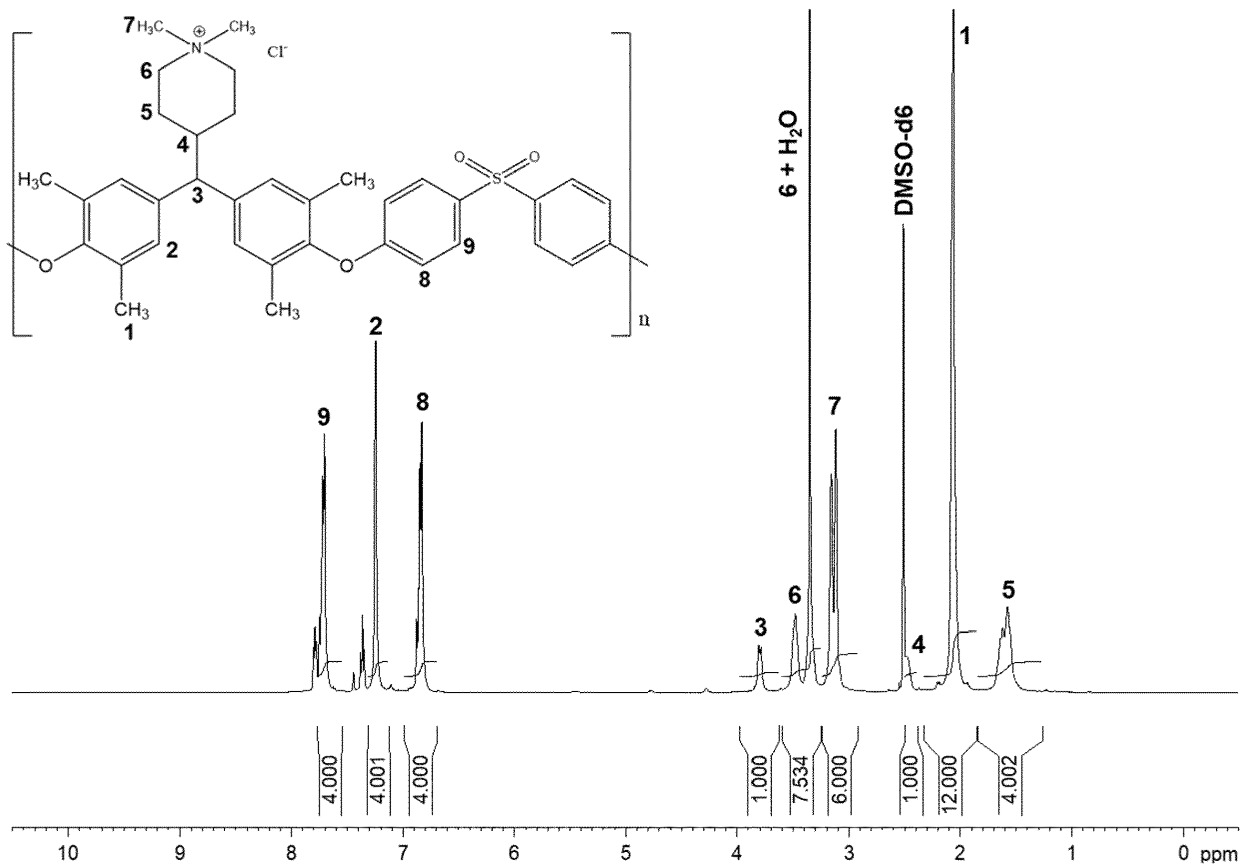
6 + $H_2O$
DMSO-d6
4.000
4.001
4.000
1.000
7.534
6.000
1.000
12.000
4.002
10
9
8
7
6
5
4
3
2
1
0 ppm

Fig. 3

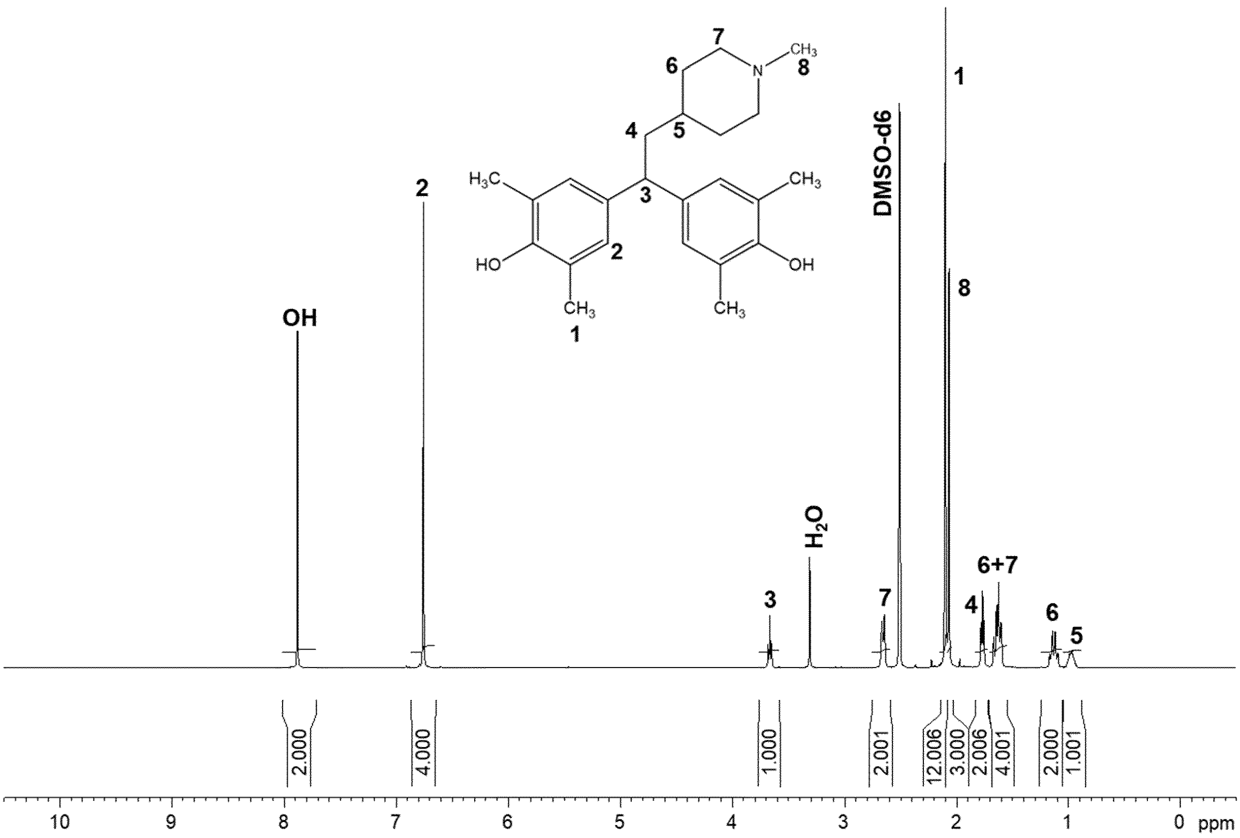
OH
2
3
$H_2O$
7
DMSO-d6
1
8
4
6+7
6
5
2.000
4.000
1.000
2.001
12.006
3.000
2.006
4.001
2.000
1.001
10
9
8
7
6
5
4
3
2
1
0 ppm

Fig. 4

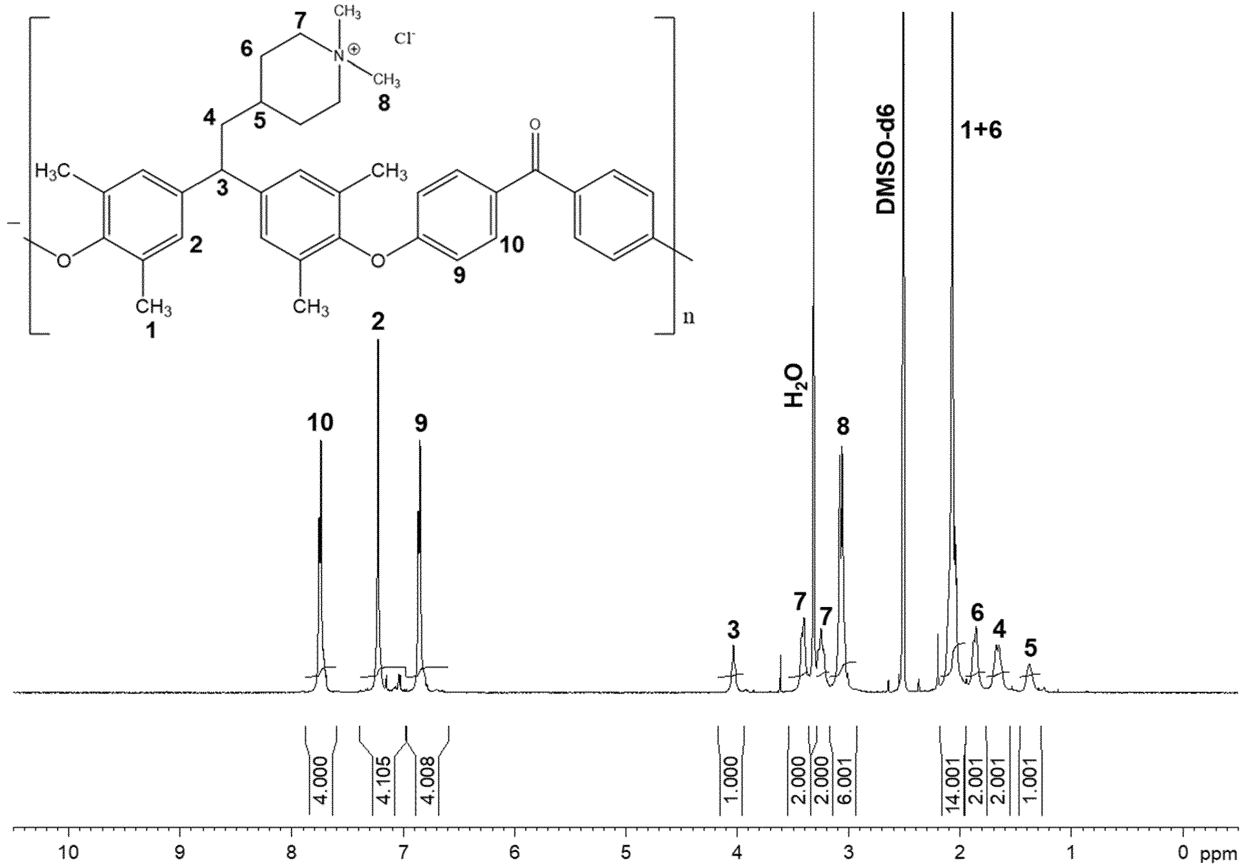

DMSO-d6
1+6
$H_2O$
10
2
9
8
7
7
3
6
4
5
4.000
4.105
4.008
1.000
2.000
2.000
6.001
14.001
2.001
2.001
1.001
10 9 8 7 6 5 4 3 2 1 0 ppm


Fig. 5

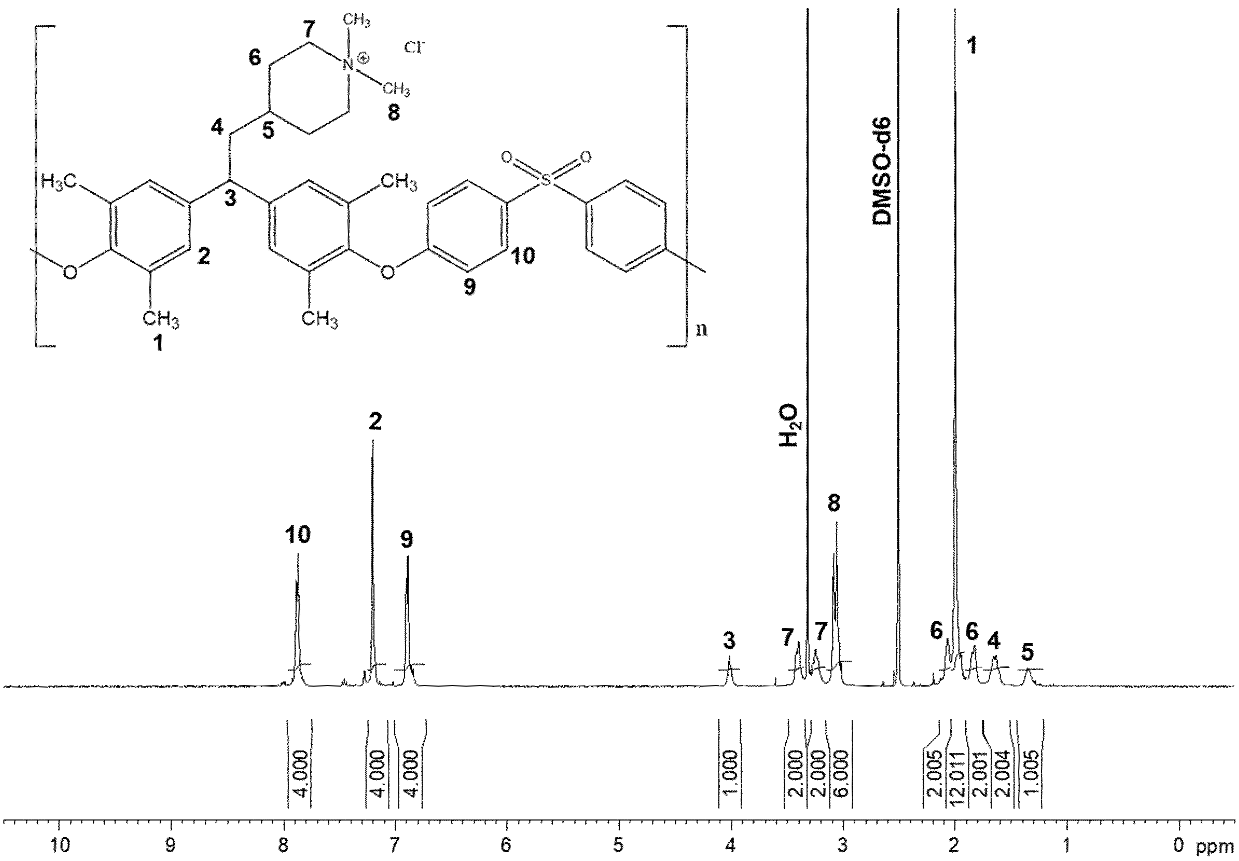

H2O
DMSO-d6
10
2
9
3
7
7
8
6
6
4
5
1
4.000
4.000
4.000
1.000
2.000
2.000
6.000
2.005
12.011
2.001
2.004
1.005
10 9 8 7 6 5 4 3 2 1 0 ppm


Fig. 6

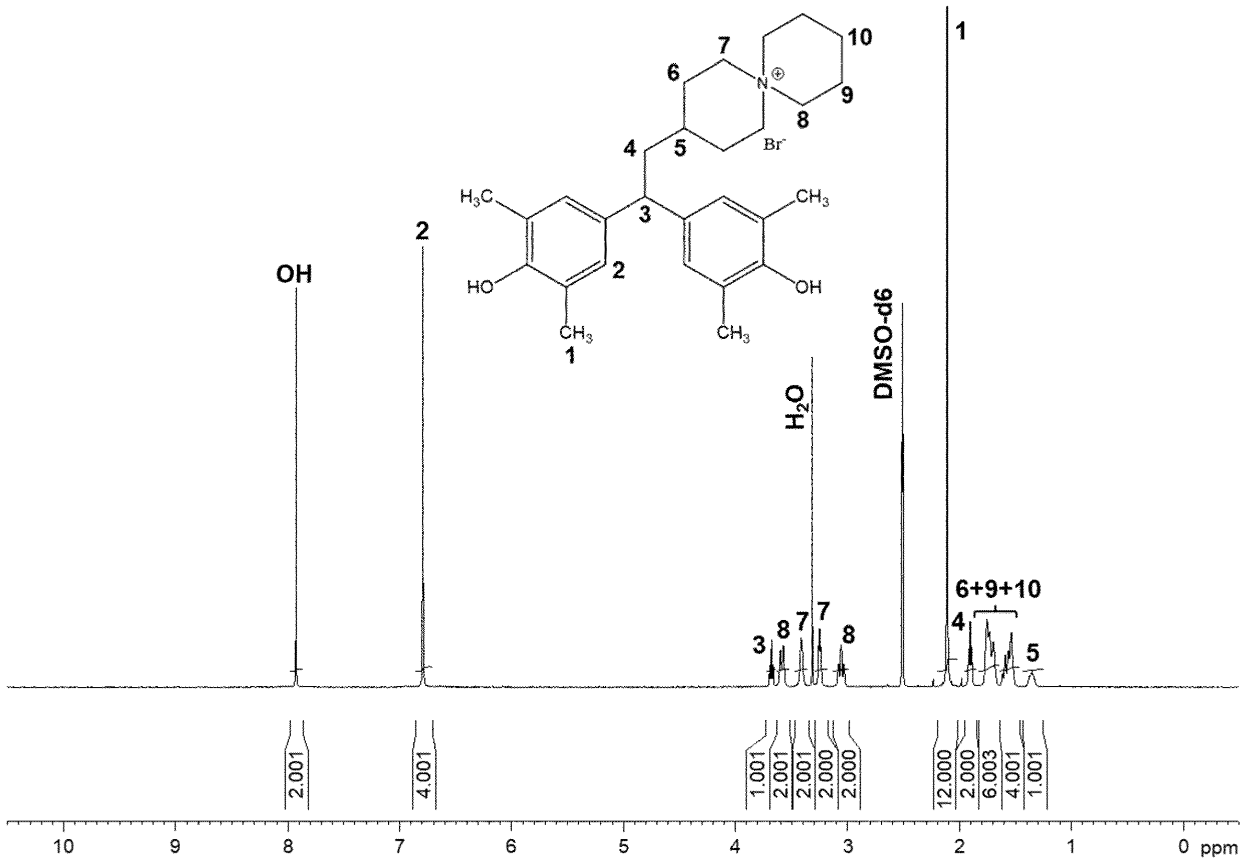
OH
2
H2O
DMSO-d6
1
3
8
7
7
8
4
6+9+10
5
2.001
4.001
1.001
2.001
2.001
2.000
2.000
12.000
2.000
6.003
4.001
1.001
10
9
8
7
6
5
4
3
2
1
0 ppm

Fig. 7

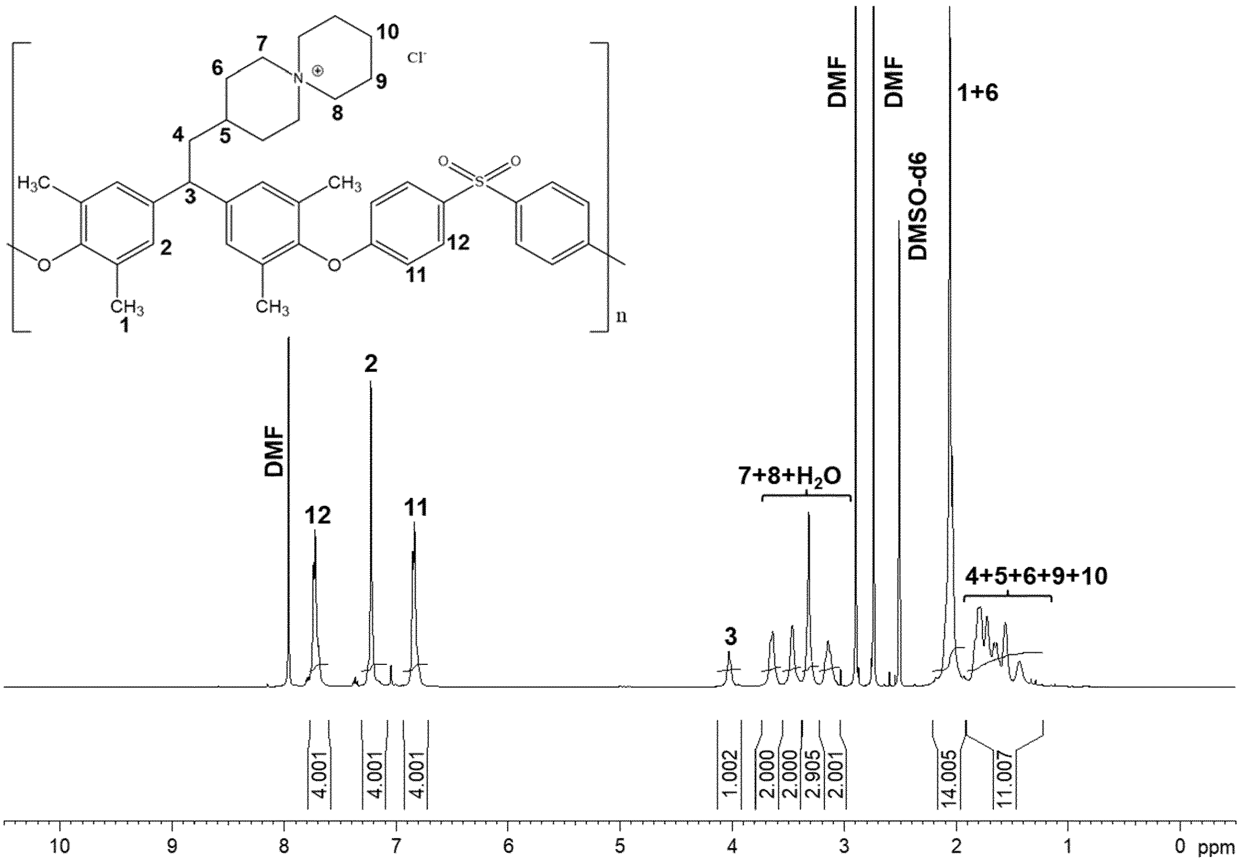
DMF
DMF
1+6
DMSO-d6
DMF
2
12
11
7+8+H$_2$O
3
4+5+6+9+10
4.001
4.001
4.001
1.002
2.000
2.000
2.905
2.001
14.005
11.007
10 9 8 7 6 5 4 3 2 1 0 ppm

Fig. 8

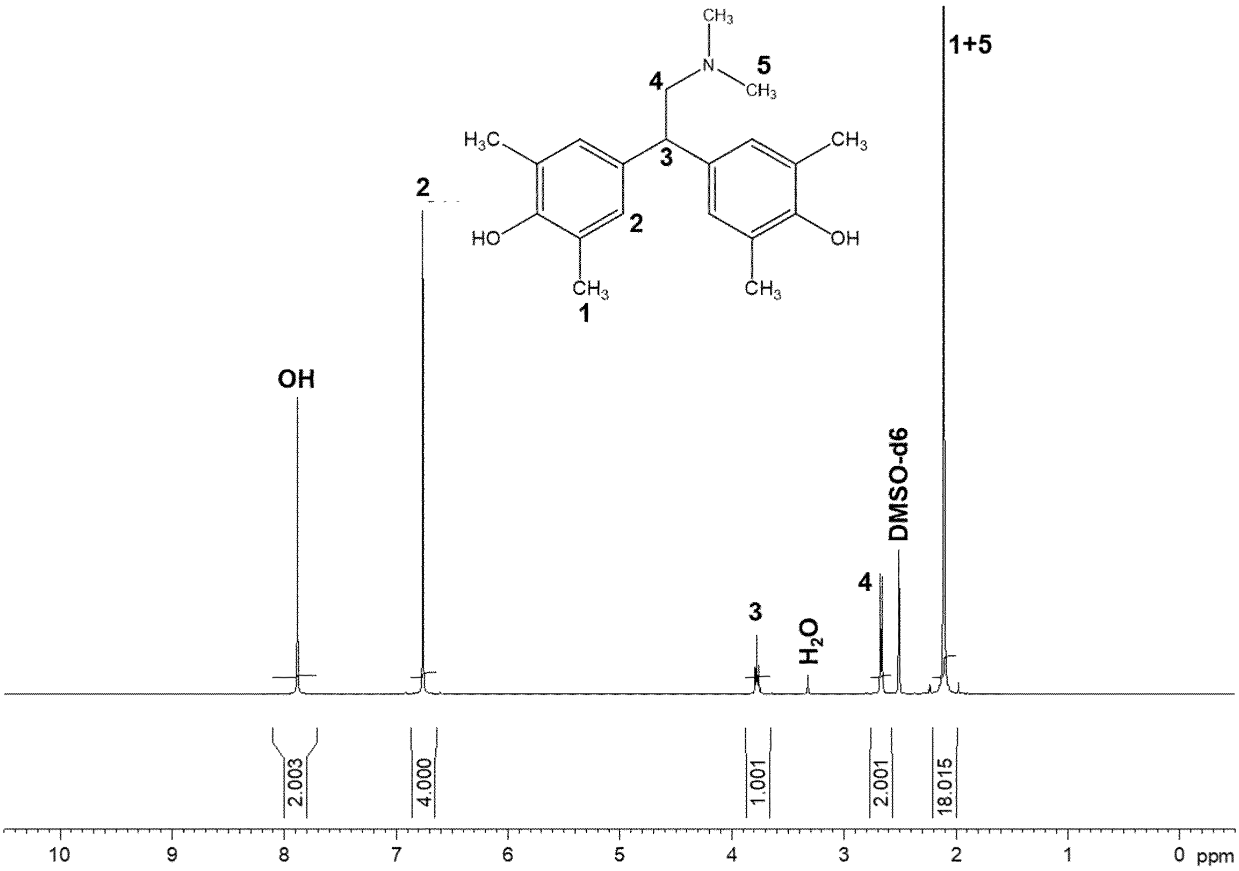
OH
2
3
$H_2O$
4
DMSO-d6
1+5
2.003
4.000
1.001
2.001
18.015
10
9
8
7
6
5
4
3
2
1
0 ppm

Fig. 9

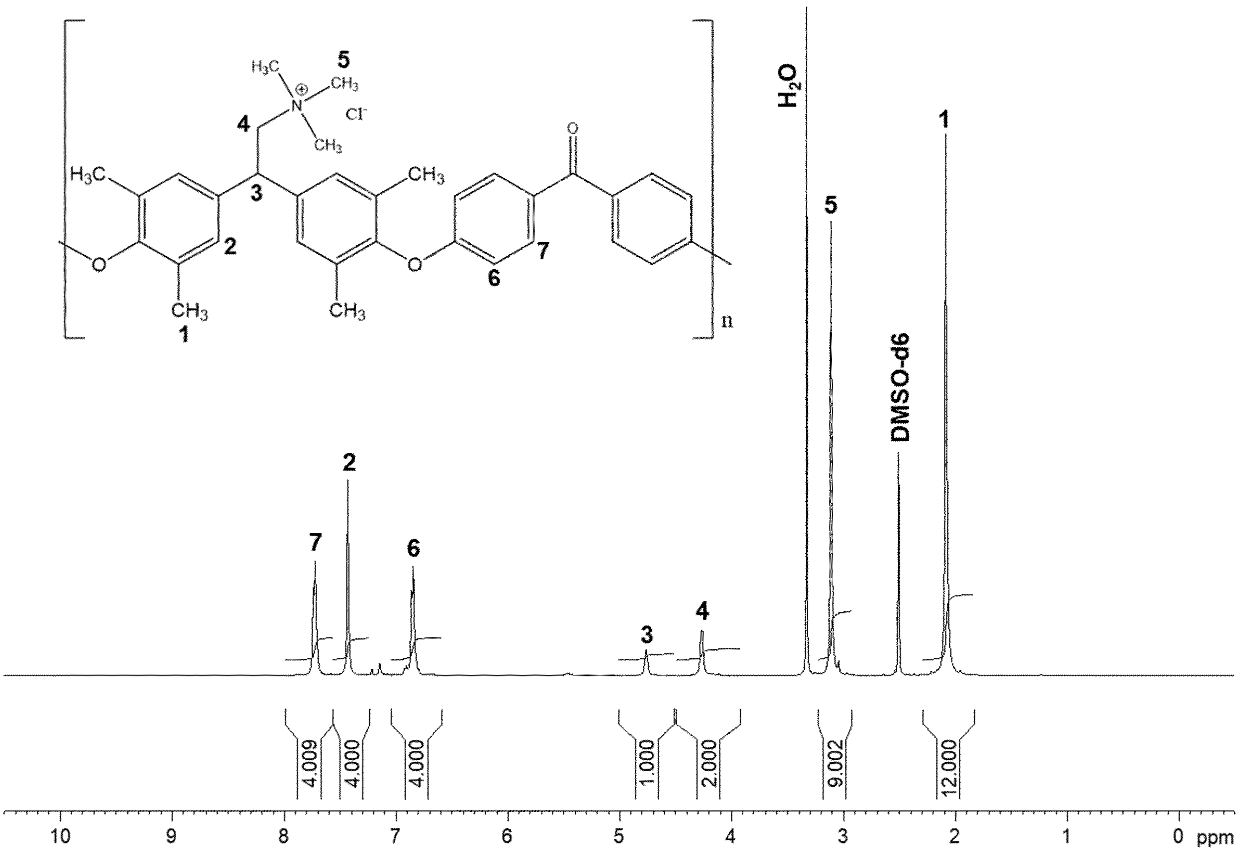
H2O
1
5
DMSO-d6
2
7
6
3
4
4.009
4.000
4.000
1.000
2.000
9.002
12.000
10 9 8 7 6 5 4 3 2 1 0 ppm

Fig. 10

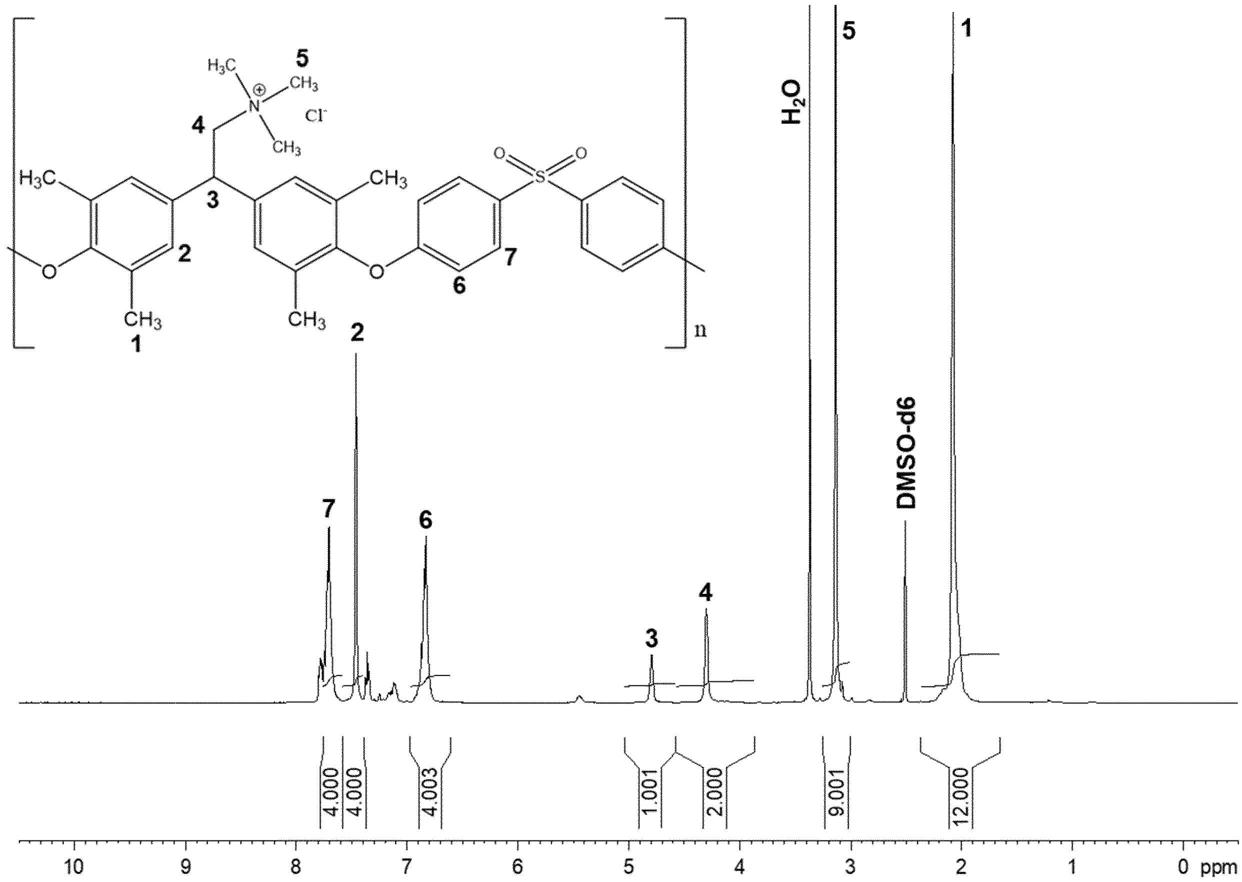
H2O
DMSO-d6
4.000
4.000
4.003
1.001
2.000
9.001
12.000
10
9
8
7
6
5
4
3
2
1
0 ppm

Fig. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 104829814 A
- CN 110294845 A
- CN 109762190 A
- WO 2019076860 A1
- WO 2021013694 A1
- EP 3770201 A1
- EP 21152487


### Non-patent literature cited in the description


- Overview: State-of-the Art Commercial Membranes for Anion Exchange Membrane Water Electrolysis. **HENKENSMEIER ; DIRK AND NAJIBAH ; MALIKAH ; HARMS ; CORINNA ; JAN ; HNÁT ; JAROMIR ; BOUZEK ; KAREL**. Journal of Electrochemical Energy Conversion and Storage. American Society of Mechanical Engineers (ASME), 2020, vol. 18, 024001
- **SU LI ; HAIBO ZHANG ; KAIQI WANG ; FAN YANG ; YUNTAO HAN ; YIRONG SUN ; JINHUI PANG ; ZHENHUA JIANG**. Anion conductive piperidinium based poly (ether sulfone): Synthesis, properties and cell performance. *Journal of Membrane Science*, 2020, vol. 594, ISSN 0376-7388, 117471, https://doi.org/10.1016/j.memsci.2019.117471
- **FEN WANG ; BOXIN XUE ; SHENGYANG ZHOU ; JIFU ZHENG ; SHENGHAI LI ; SUOBO ZHANG ; TAUQIR A**. Sherazi: Synthesis and property of novel anion exchange membrane based on poly(aryl ether sulfone)s bearing piperidinium moieties. *Journal of Membrane Science*, 2019, vol. 591, ISSN 0376-7388, 117334, https://doi.org/10.1016/j.memsci.2019.117334